# EUROPEAN PATENT SPECIFICATION

(11) **EP 1 796 737 B1**
(45) Date of publication and mention of the grant of the patent: **24.10.2018**
(21) Application number: 05773819.7
(22) Date of filing: 19.07.2005
(51) Int. Cl.: A61K 51/12, A61K 51/02, A61P 35/00, A61K 101/00, A61K 103/00, A61K 103/10, A61K 103/30, A61K 103/32, A61K 103/36

(54) **MICROSPHERES CAPABLE OF BINDING RADIOISOTOPES, OPTIONALLY COMPRISING METALLIC MICROPARTICLES, AS WELL AS METHODS OF USE THEREOF**
ZUR BINDUNG VON RADIOISOTOPEN BEFÄHIGTE MIKROKÜGELCHEN, WAHLWEISE MIT METALL-MIKROTEILCHEN UND ANWENDUNGSVERFAHREN DAFÜR
MICROSPHERES POUVANT SE LIER A DES RADIO-ISOTOPES, COMPRENANT EVENTUELLEMENT DES MICROPARTICULES METALLIQUES, ET METHODES D'UTILISATION ASSOCIEES

(30) Priority: 24.09.2004 US 613098 P
(43) Date of publication of application: 20.06.2007
(73) Proprietor: BIOSPHERE MEDICAL, INC., South Jordan, Utah 84095 (US)
(72) Inventor: KROM, James, A., Belmont, MA 02478 (US); SCHWARZ, Alexander, Brookline, MA 02446 (US)
(74) Representative: Bradley, Adrian
(86) International application number: PCT/US2005/025645
(87) International publication number: WO 2006/036269

(56) References cited:
- EP-A1- 1 371 365
- WO-A-02/34300
- WO-A-03/094930
- WO-A-2004/040972
- WO-A-2005/035005
- WO-A-2005/061009
- WO-A1-00/66183
- US-A1- 2003 120 355
- NIJSEN J F W ET AL: "Advances in nuclear oncology: microspheres from internal radionuclide therapy of liver tumors" CURRENT MEDICINAL CHEMISTRY, BENTHAM SCIENCE PUBLISHERS BV, BE, vol. 9, 2002, pages 73-82, XP002983145 ISSN: 0929-8673
- ZIELINSKI F W ET AL: "Synthesis and quality control testing of <32>P labeled ion exchange resin microspheres for radiation therapy of hepatic neoplasms" INTERNATIONAL JOURNAL OF APPLIED RADIATION AND ISOTOPES, PERGAMON PRESS LTD. OXFORD, GB, vol. 34, no. 9, 1983, pages 1343-1350, XP009082526 cited in the application
- "PHYSICAL AND CHEMICAL CHARACTERIZATION OF A POROUS PHOSPHATE-MODIFIED ZIRCONIA SUBSTRATE" JOURNAL OF CHROMATOGRAPHY, ELSEVIER SCIENCE PUBLISHERS B.V. AMSTERDAM, NL, vol. 587, no. 2, 20 December 1991 (1991-12-20), pages 137-147, XP000237101 ISSN: 0021-9673
- RAYMOND ET AL: "Production of radioactive particles for endovascular therapeutic interventions" BIOMATERIALS, ELSEVIER SCIENCE PUBLISHERS BV., BARKING, GB, vol. 27, no. 8, March 2006 (2006-03), pages 1566-1572, XP005193225 ISSN: 0142-9612

## Description

### Background of the Invention

### Embolization

Therapeutic vascular embolization procedures are used to treat or prevent certain pathological situations *in vivo.* Generally, they are carried out using catheters or syringes under imaging control to position solid or liquid embolic agents in a target vessel.

Embolization can be used to occlude partially or completely vessels of a variety of organs including brain, liver, and spinal cord. One application of embolization is to stop or reduce blood flow in hemorrhagic situations. Another application is to stop delivery of vital blood supply and nutrients to tissue; for instance, to reduce or deny blood supply to a solid tumor. In the case of vascular malformations, embolization enables the blood flow to the normal tissue, aids in surgery and limits the risks of hemorrhage. Depending on the pathological conditions, embolization can be used for temporary as well as permanent objectives.

Embolization has been performed with a variety of materials, such as small pieces of durable matters, including polyvinyl-alcohol irregular particles, geletin particles, liquid embolic products and more recently with spherical-shaped solid hydrogels. A wide variety of commercially available embolic materials are difficult to see or to trace because they are relatively transparent, cannot be seen clearly with normal light before and during administration, or are difficult to detect after administration because they are not radiopaque and lack features that render them detectable using magnetic resonance imaging, ultrasound, or nuclear medicine procedures.

### Microspheres for Embolization

U.S. Patent Nos. 5,635,215 and 5,648,100 disclose injectable microspheres comprising a hydrophilic acrylic copolymer coated with a cell adhesion promoter and a marking agent. Marking agents described in these patents include chemical dyes, magnetic-resonance-imaging agents, and contrast agents, such as barium or iodine salts. Organic dyes are complex molecules composed of aromatic structures and strong ionic charges. They are known especially in affinity chromatography as ligands for several biological structures. Their major limitation as markers for embolic agents are possible dye release as a result of the hydrolysis of the dye-embolic material link with subsequent delivery in the blood stream. Another limitation of chemical dyes is that they may be absorbed to certain biological structures or tissue, which may produce undesirable results. For example, it is well known in affinity chromatography that human albumin interacts strongly in physiological conditions with the dye Cibacron Blue F3GA.

In 1991, Thanoo *et al.* reported the preparation and properties of barium sulphate and methyl iothalamate loaded poly(vinyl alcohol) (PVA) microspheres as radiopaque particulate emboli (Journal of Applied Biomaterials, 1991, 2, 67). The barium sulphate and methyl iothalamate impregnated PVA microspheres were prepared by the glutaraldehyde cross-linking of an aqueous dispersion of PVA containing the radiopaques in paraffin oil using dioctyl sulfosuccinate as the stabilizing agent and thionyl chloride as the catalyst.

In 1998, Horák *et al.* reported radiopaque poly(2-hydroxyethyl methacrylate) (HEMA) particles containing silver iodide complexes, which were tested on cell culture (Biomaterials, 1998, 19, 1303). The incorporation of silver iodide complexes inside the poly(HEMA) particles was achieved by first swelling the particles in potassium iodide solution and precipitating the silver iodide complexes using a 30 wt% solution of silver nitrate.

Although the methods mentioned above are efficient for staining soft embolic spherical agents, such as Embosphere® or PVA microspheres, they may change the physical properties, such as density and compressibility, of the microspheres. Further, they may not provide good visibility under regular light by naked eyes for the particles before and during administration. The use of a coloring agent, such as a chemical dye, is another possibility to stain the microspheres. But the risk of this method is the release of dye molecules from the microspheres *in vivo,* as discussed above.

### Microspheres for the Treatment of Cancer

The development of new and more effective treatments for cancer is of utmost concern. This is particulary relevant for the treatment of malignant tumors found in the liver owing to unsatisfactory current treatment options. At the present time, the preferred method of treatment for patients with liver metastases is surgical resection. Unfortunately, the 5-year survival rate for patients that have undergone this form of treatment is only around 35% (Langenbeck's Arch. Surg. 1999, 313). This disappointingly low survival rate is compounded by the fact that most tumors are inoperable by the time of diagnosis. In comparison to conservative treatment, transarterial chemoembolization (TACE) has recently been shown to improve slightly the survival of hepatocellular carcinoma patients (Lancet 2002, 359, 1734). Other treatment options for these tumors include conventional chemotherapy and external radiotherapy (Int. J. Radiation Oncology Biol. Phys. 1999, 44, 189; Langenbeck's Arch. Surg. 1999, 384, 344). Unfortunately, neither of the latter regimens results in significant improvements in patient survival.

Recent developments in selective radionuclide therapy indicate that radiolabeled microspheres may offer a promising treatment option for patients suffering from a variety of types of cancer. This treatment allows the selective delivery of therapeutic radioactive particles to the tumor with as little surrounding-tissue damage as possible. This treatment option is particularly important for cancers with an extremely poor prognosis and without other adequate therapies, such as primary and metastatic malignancies of the liver. Microsphere delivery via the hepatic artery promises to be particularly effective for both primary and metastatic liver cancer since these tumors are well vascularized compared to normal liver tissue and receive the bulk of their blood supply from the hepatic artery (Surgery 1969, 66,1067); these features enable selective targeting of microspheres to the tumor tissue. In addition, many kinds of radiolabeled particles and radionuclides have been tested for local treatment of a variety of tumors in organs, including liver, lung, tongue, spleen and soft tissue of extremities.

In early applications of internal radionuclide therapy, ⁹⁰Y-containing yttrium oxide powder was suspended in a viscous medium prior to administration. Yttrium oxide was selected for the technique because it emits nearly 100 percent beta radiation (The American Surgeon 1969, 35, 181; American Surgeon 1960, 26, 678). However, the yttrium oxide powder had a high density (5.01 gm/cm³) and irregular particle shape. The high density of pure yttrium oxide powder made it difficult to keep the particles in suspension in the liquids used to inject them into the body, and the sharp corners and edges of yttrium oxide particles also irritated surrounding tissue in localized areas. In later applications, the particles used were microspheres composed of an ion exchange resin, or crystalline ceramic core, coated with a radioactive isotope, such as ³²P or ⁹⁰Y. Both ion exchange resin and crystalline ceramic microspheres offer the advantage of having a density much lower than that of yttrium oxide particles; further, the ion exchange resin offers the additional advantage of being particularly easy to label (Int. J. Appl. Radiat. Isot. 1983, 34, 1343). Microspheres have also been prepared comprising a ceramic material and having a radioactive isotope incorporated into the ceramic material. While the release of radioactive isotopes from a radioactive coating into other parts of the human body may be eliminated by incorporating the radioisotopes into ceramic spheres, the latter product form is not optimal. Processing of these ceramic microspheres is complicated because potentially volatile radioactivity must be added to ceramic melts and the microspheres must be produced and sized while radioactive, with the concomitant hazards of exposure to personnel and danger of radioactive contamination of facilities.

### Materials Used in Fabrication

Glass, resin, albumin, or polymer microspheres impregnated with a material that emits β-particles upon neutron activation have been described. The neutron activation is accomplished by subjecting the impregnated material to a high flux of thermal neutrons, usually within or near the core of the reactor. Research has indicated that the composition of the bead can be important in the design of an effective treatment. For example, glass is relatively resistant to radiation-damage, highly insoluble, and non-toxic. Glass can be easily spheridized in uniform sizes and has minimal radionuclidic impurities. Advances in manufacturing have led to the production of glass microspheres with practically no leaching of the radioactive material (Eur. J. Nucl. Med. 1997, 24, 293).

Although glass spheres have several advantages, their high density (3.29 g/ml) and non-biodegradability are major drawbacks (J. Nucl. Med. 1991, 32, 2139; Nucl. Med. Comm. 1994, 15, 545). Their relatively high density increases the chance of intravascular settling (Cancer 1998, 83, 1894). Nevertheless, glass microspheres produced under the name TheraSpheres® were the first registered microsphere product for internal radionuclide therapy, and they have been used in patients with primary or metastatic tumors.

Polymer-based microspheres have many advantages over other materials, in particular their near-plasma density, biodegradability and biocompatibility. However, the major disadvantage is their inability to withstand high thermal neutron fluxes (J. Biomed. Mater. Res. 1998, 42, 617). Sometimes additives and adjustment of irradiation-parameters can overcome this problem. A solvent evaporation technique has been used for preparation of poly(L-lactic acid) (PLLA) microspheres containing ¹⁶⁶Ho, ⁹⁰Y and ¹⁸⁶Re/¹⁸⁸Re. Mumper *et al.* has prepared PLLA microspheres with holmium-165-acetylacetonate (HoAcAc; Pharm. Res. 1992, 9, 149). HoAcAc complex and PLLA were dissolved in chloroform and the solution was added to a polyvinyl alcohol (PVA) solution and stirred until the solvent had evaporated. Microspheres were graded and collected according to size, on stainless steel sieves having 20-50 µm openings. These microspheres can be dispensed in patient-ready doses that only need to be activated by neutron bombardment to a therapeutic amount of radioactivity in a nuclear reactor. These holmium-loaded microspheres are currently being tested in intrahepatic arterial administration to rat liver tumours. A seven-fold increase of the ¹⁶⁶Ho microspheres in and around the tumor compared with normal liver was found, based on distribution of radioactivity.

An alternative approach for preparing radioactive polymer-based microspheres is to contact the polymer with a radioisotope, rather than by neutron activation of a polymeric material impregnated with a nonradioactive precursor isotope. The radioactivity may be incorporated during or after the fabrication of the polymer into microsphere form. Polymeric ion exchange resins are commonly employed for this purpose. Chloride salts of holmium and yttrium have been added to cation exchange resins. Different resins were investigated by Schubiger *et al.,* amongst which were Bio-Rex 70, Cellex-P, Chelex 100, Sephadex SP and AG 50W-X8 (Nucl. Med. Biol. 1991, 18, 305). The resins with ⁹⁰Y bound to the carboxylic acid groups of the acrylic polymer were sterilized and used for renal embolization of pigs. Only the pre-treated Bio-Rex 70 resulted in usable particles, with a retention of beta activity in the target organ of >95% of injected dose, and no histologically detectable particles in lung tissue samples (Invest. Rad. 1995, 30, 716).

Aminex resins (Bio-Rad Inc. Hercules CA, USA) loaded with ¹⁶⁶Ho or ¹⁸⁸Re also resulted in usable preparations. Turner *et al.* prepared microspheres by addition of ¹⁶⁶Ho-chloride to the cation exchange resin Aminex A-5, which has sulphonic acid functional groups attached to styrene divinylbenzene copolymer lattices (Nucl. Med. Comm. 1994, 15, 545). Reproducible, non-uniform distributions of the ¹⁶⁶Ho-microspheres throughout the liver were observed on scintigraphic images, following intrahepatic arterial administration in pigs. This predictable distribution allowed these investigators to determine the radiation absorbed dose from a tracer activity of ¹⁶⁶Ho-microspheres, and to define the administered activity required to provide a therapeutic dose. Aminex A-27 was labelled with ¹⁸⁸Re by adding ¹⁸⁸Re-perrhenate and SnCl₂ to vacuum-dried resin particles (J Nucl. Med. 1998, 39, 1752). The mixture was boiled and centrifuged and microspheres were separated and resuspended in saline. Spheres were tested by direct intratumoural injection into rats with hepatoma. Survival over 60 days was significantly better in the treated versus the control group (80% vs. 27%). An example of a ⁹⁰Y-coated ion exchange resin is described in WO 02/34300; it is believed that the composition and methods described therein are currently marketed under the trade designation SIRspheres by Sirtex Medical Limited (New South Wales, Australia).

Investigators from Australia and Hong Kong have used unspecified resin-based particles labeled with ⁹⁰Y for treatment of patients with primary or secondary liver cancer (Br. J. Cancer 1994, 70, 994). The spheres had a diameter of 29-35 µm, a density of 1.6 g/mL and a specific activity of approximately 30-50 Bq per sphere. Treatment was well tolerated with no bone-marrow or pulmonary toxicity. The median survival was 9.4 months (range 1.8-46.4) in 71 patients, and the objective response rate in terms of drop in tumour marker levels was higher than that based on reduction in tumor volume shown by computed tomography (Int. J. Radiation Oncology Biol. Phys. 1998, 40, 583).

In another instance, magnetic PLLA microspheres loaded with yttrium-90 were made by Hafeli *et al.* in order to direct them to the tumor (Nucl. Med. Biol. 1995, 22, 147). This method resulted in stably loaded spheres, with the possibility of pre- or afterloading. To produce preloaded microspheres, PLLA was dissolved with L-α-phosphatidylcholine in methylene chloride. Commercially available ⁹⁰YCl₃ and magnetite Fe₃O₄ were added to the solution, vortexed, and sonicated. The suspension was injected into PBS with PVA, and microspheres were prepared following a solvent evaporation technique. After-loaded spheres were prepared by suspending dried microspheres in a solution of PBS, to which ⁹⁰YCl₃ in HCl was added. Spheres were subsequently vortexed, incubated, and washed, resulting in labeled microspheres. Leaching of ⁹⁰Y was around 4% after 1 day in PBS at 37°C. Specific activity was 1.85 MBq/mg in both methods. ⁹⁰Y was bound to the carboxylic acid groups of the PLLA. Experiments in mice showed a 12-fold increase in activity in the tumor with a directional magnet fixed above it. Rhenium-loaded PLLA microspheres were also developed, but these microspheres were unable to withstand the high neutron fluxes in a nuclear reactor which are necessary to achieve the high specific activity required in the treatment of liver tumors (Int. J. Radiation Oncology Biol. Phys. 1999, 44, 189).

The development of microspheres for radionuclide therapy is further complicated by the difficulty in determining the biodistribution of the microspheres in vivo, as noted above for ⁹⁰Y. The biodistribution of microspheres is critically important for this type of radiotherapy because the microsphere must be in close proximity to the tumor being treated. One potential solution to this problem would be to associate a material with the microsphere that is capable emitting a detectable, non-hazardous signal, which would allow the determination of the radiation dose distribution in the tissue. Thus, any tumor tissue that escaped effective radiotherapy ("cold spots") could be detected, which would indicate retreatment. An example of such a signal is a γ-photon of appropriate energy. Radioisotopes that emit γ-photons suitable for diagnostic imaging include ^{99m}Tc, ¹¹¹In, ⁶⁷Ga, and ²⁰¹Tl.
The document WO 2004/040972 A2 discloses polymeric materials that are labelled with colloidal gold. The document WO 03/094930 A discloses a method of temporary embolization using a hydrogel which only hydrolizes at a specific pH. The document EP 1371365 A1 discloses methods for forming polymeric microspheres having narrow particle size distributions. The document WO 00/66183 discloses an embolic material comprising a hydrophilic polymer and a metal. The document "Advances in Nuclear Oncology: Microspheres for Internal Radionuclide Therapy of Liver Tumours", Nijsen et al. Current Medicinal Chemistry 2002, 9, 73-82, discloses the preparation, stability and degradation of various radiolabelled microspheres. The document "Synthesis and Quality Control Testing of 32P Labelled Ion Exchange Resin Microspheres for Radiation Therapy of Hepatic Neoplasms", Zielinski et al. Int. J. Appl. Radiat. Isot. Vol. 34, No. 9, pp. 1343-1350. 1983, discloses the synthesis of ³²P labelled microspheres. The document US 2003/0120355 A1 discloses polymers which are able to bind radioisotopes.

### Summary of the Invention

One aspect of the present invention relates to a microsphere according to claim 1. In certain embodiments, the microsphere further comprises a second radioisotope; wherein the atomic number of the first radioisotope is not the same as the atomic number of the second radioisotope.

In further aspects the present invention also provides a methods of preparing a radioactive microsphere according to claims 12, 17 and 20.

Further aspects of the present invention relate to microspheres for the treatment of a mammal according to claim 25, and for treatment according to claim 26.

Also provided is a composition for use in a method of treatment of a mammal suffering from a medical condition, comprising the step of administering to said mammal a composition containing therapeutically effective amount of radioactive microspheres as defined in the claims comprising a hydrophilic polymer and a first radioisotope. In certain embodiments, the microsphere further comprises a second radioisotope; wherein the atomic number of the first radioisotope is not the same as the atomic number of the second radioisotope.

Also disclosed is a microsphere, comprising a hydrophilic polymer comprising a plurality of pendant moieties; an insoluble transition-metal, lanthanide or group 13-14 oxide, polyoxometalate, hydroxide, alkoxide, carboxylate or combination thereof; and a first radioisotope. In certain embodiments, the microsphere further comprises a second radioisotope; wherein the atomic number of the first radioisotope is not the same as the atomic number of the second radioisotope.

Also disclosed is a method of preparing a radioactive microsphere, comprising the steps of: 1) fabricating a microsphere, 2) contacting the microsphere with at least one soluble transition-metal, lanthanide or group 13-14 compound or salt, 3) converting said transition-metal, lanthanide or group 13-14 compound or salt into an insoluble form, and 4) associating the resulting transition-metal, lanthanide or group 13- 14 impregnated microsphere with a radioisotope; optionally associating said microsphere with additional radioisotopes. Optionally, step 2 can be omitted by fabricating the microsphere in the presence of a soluble transition-metal, lanthanide or group 13-14 compound or salt. Optionally, steps 2 and 3 can be omitted by fabricating the microsphere in the presence of a suspension or colloid of an insoluble transition-metal, lanthanide or group 13-14 compound or salt.

### Detailed Description of the Invention

The invention will now be described more fully with reference to the accompanying examples, in which certain preferred embodiments of the invention are shown.

### Overview of a Preferred Embodiment

According to the present invention, the metal-compound-containing polymeric materials ("composite materials") may be used in any medical applications, but they are especially suitable as implantable and/or injectable devices. In certain embodiments of the present invention, the composite material is in microparticle form and is useful as emboli for prophylactic or therapeutic embolizations. Therefore, the composite materials of the present invention are particularly suitable in injectable implantations or embolizations as small particles, such as microparticles, microbeads or microspheres. These microparticles are usually difficult to detect after injection into the body. In certain embodiments of the present invention, the microparticles are rendered detectable by associating them with a suitable γ-emitting radioisotope.

Radionuclide therapeutic techniques using microspheres for the treatment of various ailments rely upon the precise and accurate delivery of microspheres to a target. This treatment option offers the promise of delivering therapy directly to the afflicted area, minimizing damage to nearby healthy tissue, a serious shortcoming associated with conventional treatment options, such as chemotherapy, radiotherapy, or surgical resection. However, the effectiveness of treatments using radionuclide microspheres would be improved by their formulation at the point of use (e.g., at a hospital's radiopharmacy). This would allow physicians to prescribe customized doses of radiation to a patient. Therefore, a microsphere that could be associated with a radioactive isotope at the point of use would be highly desirable. Microspheres comprising a hydrophilic polymer comprising a plurality of pendant moieties; comprising zirconium oxide, polyoxometalate, hydroxide, alkoxide, carboxylate or combination thereof; have been designed to associate with a radioisotope being in the form of ³²P phosphate that emits a therapeutic .beta.-particle. In certain embodiments, the microsphere further comprsies a second radioisotope that emits a diagnostic γ-ray; wherein the atomic number of the first radioisotope is not the same as the atomic number of the second radioisotope.

### Bulk Composition of Microspheres

A microsphere of the present invention can be fabricated from any hydrophilic polymer. The polymeric material of the present invention includes natural and synthetic polymers. Preferably, the natural polymer or derivative thereof comprises crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, or related polymers. In certain embodiments of the present invention, the material comprises one or more polymers selected from the group consisting of acrylics, vinyls, acetals, allyls, cellulosics, methacrylates, polyamides, polycarbonate, polyesters, polyimide, polyolefins, polyphosphates, polyurethanes, silicones, styrenics, and polysaccharides. In certain embodiments one or more of the polymerized monomers is selected from the group consisting of acrylate, methacrylate, ethylene glycol methacrylate phosphate, and vinylphosphonate. In certain embodiments, the polymeric material of the present invention is or is made to be an elastomer, a hydrogel, a water-swellable polymer, or combinations thereof. All of these polymers are preferably crosslinked so as to be insoluble.

In certain embodiments of the present invention, the hydrogel microsphere comprises a polymeric material that comprises a hydrophilic copolymer, which contains, in copolymerized form, about 1 to about 15%, by weight, of a difuinctional monomer and about 85 to about 99%, by weight, of one or more hydrophilic monomers. More preferably, the hydrophilic monomer is selected from the group consisting of acrylate, methacrylate, ethylene glycol methacrylate phosphate, vinylphosphonate, and N-[tris(hydroxymethyl)methyl]acrylamide; the difunctional monomer is selected from the group consisting of N,N'-methylenebisacrylamide, N,N'-diallyltartardiamide, and glyoxal-bis-acrylamide. In a most preferred embodiment of the present invention, the polymeric material comprises a copolymer of vinylphosphonate, N-[tris(hydroxymethyl)methyl] -acrylamide, and N,N'-methylenebisacrylamide.

In certain embodiments of the present invention, the polymeric materials comprise pendant metal-complexing moieties. These moieties can be in their conjugate base form at around physiological pH. Preferably, these moieties are selected independently from the group consisting of phosphonic acids, bisphosphonic acids, phosphates, polyphosphates (including diphosphates and triphosphates), sulfonic acids, sulfates, carboxylic acids, carbamic acids, hydroxamic acids, acyl hydrazides, thiols, amines, silicates, aluminates, titanates, zirconates, and heterocycles such as pyridine, imidazole, thiophene, thiazole, furan, purine, pyrimidine, hydroxyquinoline and metal-complexing dyes. Most preferably, these functional groups are phosphonic acids, phosphonates, phosphates, bisphosphonic acids, bisphosphonates, or polyphosphates. In certain embodiments, the metal-complexing moiety can be introduced into the polymer in a separate step (*e.g.,* by a grafting reaction).

In certain embodiments of the present invention, the polymeric materials comprise microbeads or microparticles based on a biocompatible, hydrophilic, substantially spherical, and non-toxic polymers. The microspheres are injectable and/or implantable and are not susceptible to digestion or elimination through the mammal's immune, lymphatic, renal, hepatic, pulmonary, or gastrointestinal system or otherwise. In other embodiments of the invention, the microspheres can be eliminated by the mammal.

The polymeric material of the present invention optionally contains zirconium oxides, hydroxides, alkoxides, carboxylates, or combinations thereof, that have dimensions ranging from about 1 µm to about 2000 µm, more preferably, from about 1 µm to about 100 µm, most preferably, from about 10 µm to about 40 µm.
A metal or metal compound, the metal being zirconium, is used in the present invention. Further metals or metal compounds can be included, as described below. The further metal or metal compounds can be, e.g. scandium, yttrium, lanthanum, hafnium, titanium, aluminum, silicon, gallium, indium, thallium, germanium, tin, lead, bismuth, tungsten, tantalum and/or any of the lanthanides (rare earths). The association of the particles of metals or inorganic metal compounds within the polymers is the result of either direct deposition of the particles on the porous polymeric material or a precipitation, reduction or oxidation process from a metal salt solution (*e.g.,* a solution of metal halides, sulfonates, carboxylates, nitrates, or alkoxides) or a combination of any of them. Alternatively, the association of the particles of metals or inorganic metal compounds within the polymers is accomplished by performing the polymerization in the presence of a metal-containing solution, suspension, or colloid.

The microsphere of the present invention may also comprise one or more cell adhesion promoters selected from the group consisting of collagen, gelatin, glucosaminoglycans, fibronectin, lectins, polycations, natural biological cell adhesion agents and synthetic biological cell adhesion agents. Further, the microsphere may optionally comprise a marking agent selected from the group consisting of dyes, imaging agents, and contrast agents.

### Certain Processes for Associating Polymeric Materials with Metal Particles

Also disclosed are processes of associating transition-metal, lanthanide or Group 13-14 metal particles with polymeric material. In the present invention, the transition-metal is zirconium and it is present in the form of an oxide, polyoxometalate, hydroxide, alkoxide, carboxylate or combination thereof in the claimed microspheres. The association process can be accomplished in at least three ways. First, the particles can be associated with, or precipitated in the pores of, the polymeric materials via a chemical reaction. Second, the particles can be deposited on and/or within the polymeric material through direct contact between the material and a colloidal solution or suspension of the particles. Third, the metal-containing polymeric material can be produced by introducing a metal salt solution, suspension, or colloid into the initial polymerization solution or suspension of the polymeric material. In all three methods, the metal particles are preferably permanently associated with the polymeric materials or within the pores thereof, enabling better detection and control of such materials in implantation applications. The various polymeric materials mentioned above are suitable for the association processes.

Herein is also disclosed that transition-metal, lanthanide or Group 13-14 metal particles can be associated with a polymeric material by contacting the polymeric material with a metal salt solution for a time and at a temperature sufficient to bind, associate, reduce, oxidize, or precipitate the metal salt into metal-containing particles that are deposited on or within the polymeric material. In certain processes the polymeric material is porous and the process enables the porous materials to comprise at least part of the metal particles within the pores of the material. In such cases, the sizes of the metal particles may either be larger or smaller than the sizes of the pores of the material, as measured by the cross-sections of the pores.

In certain processes, the metal salt solution is zirconium acetate (in aqueous acetic acid) having a concentration ranging from about 0.1% to about 20% by weight of zirconium. The microspheres are subsequently washed with water and treated with dilute aqueous ammonia. The product is obtained by washing the microspheres with water. The product can be sterilized, for example, by autoclaving.

Also disclosed is a process of associating metal particles or metal compounds with a polymeric material by contacting the polymeric material with a colloidal solution of said particles. In certain processes, the polymeric material is porous and the process enables the porous materials to comprise at least part of the metal particles within the pores of the material. In such a process, the sizes of the metal particles are preferably smaller than the sizes of the pores, as measured by the dimension of the cross sections of the pores.

A preferred process for this direct deposition of metal particles comprises packing the polymeric material, such as microparticles, in a column and perfusing the column with the metal solution. This process can be preferably followed by rinsing the column with water or saline. When colloidal particles are used for porous materials, the particles are preferably of sizes smaller than the pores of the polymeric material. They also are preferably suspended with a surfactant to minimize or eliminate aggregation.

Another process of associating particles of metal or metal compound with the polymeric material comprises adding the metal particles or their corresponding salt solution or colloid into the initial polymerization solution or suspension for the polymeric material. In another process, the resultant polymeric material is porous and the process enables the porous materials to comprise at least part of the metal particles within the pores of the material.

In such a polymerization/association process, there is preferably no change in the polymerization process for the polymeric material itself. Therefore, any polymerization process that produces a polymeric material can be incorporated into the process by adding a metal salt solution, colloid, or suspension into the initial polymerization solution or suspension. In particular, polymerization processes disclosed in U.S. Pat. No. 5,635,215 for producing acrylic microspheres and in WO 00/23054 for producing PVA microspheres can be incorporated into the process to produce hydrophilic acrylic microspheres or PVA microspheres containing colloidal particles. When the initial polymerization solution or suspension is transformed into an acrylic or PVA microsphere, preferably in hydrogel form, the colloidal particles are trapped within the polymer network and can no longer be released to a substantial extent. In this case they are located inside the polymer pores. In the case of a porous polymeric material, the resulting metal-containing material from this process may contain colloidal metal particles that are larger than the sizes of the pores, as measured by the dimensions of the cross sections of the pores. However, in practice, if the affinity of the metal for the monomer is too high, then precipitation will occur faster than polymerization (as is the case with the polymerization of vinylphosphonate in the presence of zirconium acetate).

### Identity of β-Emitting Therapeutic Radionuclide

Only a few radioisotopes have desirable characteristics necessary for the diagnostic or therapeutic treatment of tumors. Important characteristics of a suitable radioisotope include a radiational spectrum (energy distribution of radiation emission) appropriate to the size of the tumor, high dose rate, and short half-life. Additionally, in the case of diagnostic treatment or dosimetric measurements, a suitable γ-emission is necessary for external imaging.

A radionuclide suitable for internal radionuclide therapy will preferably have a number of characteristics. First, the radioisotope must have an appropriate radiation spectrum. Second, a high dose rate is advantageous for the radiobiological effect (Nucl. Med. Biol. 1986, 13, 461; Nucl Med. Biol. 1987, 14, 537). Consequently, a short half-life is preferable. Finally, for this application, the radioisotope must have an affinity for the composite microsphere. This affinity may be conferred by incorporating the radioisotope in a suitable chemical species, such as a complex ion or colloid. The present invention involves a radioisotope in the form of ³²P phosphate. A reference example of a radioisotope in a colloidal form is ¹⁸⁶Re complexed with tin, which can be prepared, for example, by treating an aqueous solution of ¹⁸⁶Re-perrhenate with SnCl2. Certain other radioisotopes in their simple ionic forms may possess sufficient affinity for the microsphere; another reference example is ⁹⁰Y as its 3+ ion. It is desirable, but not necessary, that the β-emitting radioisotope also emits γ photons that are detectable, for example, by gamma camera, for imaging purposes. Unfortunately, only a few β-emitting radioisotopes have characteristics which make them potentially suitable for use. Reference radionuclides are selected from the group consisting of the lanthanides, yttrium, strontium, gold, phosphorus, and iridium. Radioactive palladium (¹⁰³Pd) and ytterbium (¹⁶⁹Yb) are also contemplated, although they emit soft x-rays, rather than β particles. In miscrospheres falling outside the scope of the invention, the radionuclide may be ⁹⁰Y, ¹⁴⁰La, ¹⁵³Sm, ¹⁶⁵Dy, ¹⁶⁶Ho, ¹⁶⁹Er, ¹⁷⁵Yb, ¹⁷⁷Lu, ¹⁸⁶Re, ¹⁸⁸Re, or combinations thereof.

In the invention, the radionuclide is ³²P phosphate, due to its advantageous physical properties and its ability to bind tightly to metal oxides, such as zirconia (J. Chromatogrpahy 1991, 587, 137). The maximum energy of the ³²P *β*-particle is 1.71 MeV resulting in a maximum range in tissue of about 8 mm. The average range is 2 mm. This range is short enough to minimize unwanted irradiation of sensitive adjacent organs; radiation hazards to operating personnel are likewise minimized. However, the range is sufficiently great to give an acceptable radiation dose distribution when the microspheres are uniformly distributed in well-perfused tissue. The radiation dose (rads) from ³²P is 733 times the tissue concentration (µCi/g). The half life is 14.3 days giving great flexibility in scheduling synthesis, quality control, and administration. Finally, ³²P phosphate is readily available worldwide at low cost.

In related applications, the use of ³²P-containing glass and ceramic microspheres has been reported; however, these microspheres utilized ³¹P as a precursor, which necessitated the bombardment of said microspheres with neutrons at a nuclear reactor to form ³²P. Unfortunately, they suffered from the formation of unwanted radioisotopes, leaching of the ³²P, and required other expensive and difficult processing steps (Nuc. Med. Biol. 1987, 14, 233 and references therein). An earlier report by Zielinski and Kasprzyk discussed the formation of cation exchange resin beads which were treated with chromium and associated with ³²P phosphate. (Int. J. Appl. Radiat. Isot. 1983, 34, 1343). This composition suffered from various drawbacks. Although not explicitly described by the authors, it is believed that the cation exchange resin (AG 50W x 12, supplier not specified) comprised anionic sulfonate moieties. By comparison, chromium sulfate is appreciably water soluble, so it can be reasonably expected that the chromium would be reversibly bound to the resin. In fact, cation exchange resins are designed so that various cations reversibly bind to them, and can be displaced by other cations, such as sodium from aqueous sodium chloride. Indeed, the authors state that the microspheres are not stable in boiling saline, and a stabilization step was therefore required after labeling the microspheres with radiophosphate. Chromium, furthermore, is a known toxin, and the authors did not demonstrate that these microspheres are nontoxic. In contrast, the microspheres of the present invention are nontoxic, and do not require chemical stabilization after labeling with the radioisotope. In another reference embodiment, the radionuclide is ⁹⁰Y, which also possesses an advantageous beta-emission spectrum. The maximum energy of the ⁹⁰Y *β*-particle is 2.27 MeV resulting in a maximum range in tissue of about 11 mm, and an average range of about 3.6 mm. The half life is only about 64 hours, resulting in a high radiation dose rate.

Compositions comprising ⁹⁰Y-coated ion exchange resins have been reported (WO 02/34300). Commercial ion exchange resin was treated with an aqueous solution of ⁹⁰Y yttrium sulfate solution, followed by contacting the resin with sodium phosphate to precipitate yttrium on the surface or in the pores of the resin. Here again, stabilizing the radiolabeled composition is required, but the stabilization is provided by phosphate precipitation. The chemistry in this application is in fact similar to that described above for the chromium-containing ion exchange resin, viz, a metal is introduced into a sulfonated ion exchange resin, followed by precipitation of the metal with phosphate, In the chromium example, the phosphate is radioactive; in the yttrium example, the metal is radioactive.

Traditionally, the most suitable β-emitting radioactive materials have been phosphorus-32, yttrium-90, rhenium-188, holmium-166. All three of these materials emit β-radiation useful for radiotherapy. Although ⁹⁰Y is often used in radionuclide therapy, yttrium-90 has two major disadvantages for use in radiotherapy. First, long neutron activation times (>2 weeks) are needed to achieve therapeutic activities of yttrium because the precursor of ⁹⁰Y has a small thermal neutron cross section of 1.28 barn. Secondly, the biodistribution of microspheres loaded with ⁹⁰Y cannot be directly determined in clinical trials because ⁹⁰Y is a pure β-emitter, i.e., it does not produce imageable γ-rays. Natural rhenium is composed of two isotopes, ¹⁸⁵Re and ¹⁸⁷Re, that form β-emitting ¹⁸⁶Re and ¹⁸⁸Re radioisotopes, respectively, upon neutron activation. The nuclear and dosimetric properties of the rhenium radioisotopes are comparable to those of ⁹⁰Y, but they have imageable γ-photons. Like the rhenium radioisotopes, ¹⁶⁶Ho emits β-particles and γ-photons and has a relatively short physical half-life of 26.8 h, compared to ⁹⁰Y (64.1 h) and ¹⁸⁶Re (90.6 h), resulting in a high dose rate.

### Detection of γ Photons Emitted by Diagnostic Radionuclide

Today, cancer is often found using a gamma camera, which provides images of potential tumors in the body by detecting the radiation emitted by a radiopharmaceutical given to a patient undergoing a full-body scan. In such systemic approaches, suspected tumor regions collect higher concentrations of the radiopharmaceutical, which produces a higher count rate and, therefore, a detectable contrast between the tumor region and its surroundings.

Most clinically-used radiopharmaceuticals are diagnostic agents incorporating a gamma-emitting nuclide which, because of physical or metabolic properties of its coordinated ligands, localizes in a specific organ after intravenous injection. The resultant images can reflect organ structure or function. These images are obtained by means of a gamma camera that detects the distribution of ionizing radiation emitted by the radioactive molecules. The principal isotope currently used in clinical diagnostic nuclear medicine is technetium-99m, which has a half-life of 6 hours. In the radiopharmacy, technetium is invariably collected from a generator as pertechnetate ion, and pertechnetate is expected to have little or no affinity for the composition of the present invention. Since the degree to which the isotope can bind to the metal-containing microsphere is of most importance, technetium-99m, indium-111, gallium-67, and thallium-201 (as their cations), and ¹⁸F (as its anion) are among the suitable gamma emitters. ¹⁸F does not emit gamma photons directly; rather, it emits positrons, which react with surrounding electrons to generate gamma photons. The use of technetium is expressly contemplated, but an extra processing step may be required, such as reduction of pertechnetate with SnCl₂, given the relatively low affinity of pertechnetate for the composition described herein.

As outlined above, a gamma camera is used in nuclear medicine for the display, in an organ, of the distribution of molecules marked by a radioactive isotope injected into a patient. Thus, a gamma camera has a collimator to focus the gamma photons emitted by the patient's body, a scintillator crystal to convert the gamma photons into light photons or scintillations, and an array of photomultiplier tubes, each of which converts the scintillations into electrical pulses. Such a detection system is followed by a processing and display unit that can be used to obtain an image projection of the distribution of the radioactive isotopes in the patient during the acquisition of the image.

### Processes & Advantages of Associating Composite Microspheres with Radioisotopes

Importantly, the microspheres of the present invention can readily be labeled with radioactivity at the point of use (*e.g.,* at a hospital's radiopharmacy). This characteristic will allow physicians to prescribe customized doses of radiation to the patient. The microspheres of the present invention are radiolabeled with ³²P-phosphate. The microspheres can be further radiolabeled with an isotope intended for therapeutic purposes (*e.g.*, yttrium-90) and/or imaging purposes (*e.g.,* technetium-99m, indium-Ill or gallium-67). The microspheres herein described strongly absorb these species from solution, facilitating proper dosing and minimizing undesirable radioactive waste.

### Administration of Microspheres

The microspheres may be administered to the patient through the use of syringes or catheters either alone or in combination with vasoconstricting agents or by any other means of administration that effectively causes the microspheres to become embedded in the cancerous or tumor-bearing tissue (U.S. Pat. No. 5,302,369). For purposes of administration, the microspheres are preferably suspended in a biocompatible fluid medium. More preferably, said medium has a sufficient density or viscosity that slows or prevents the microspheres from settling out of suspension during the administration procedure. Most preferably, said medium is also sufficiently opaque to be detectable by x-ray imaging (i.e., radiopaque) to allow visualization of the injection. Presently, preferred liquid vehicles for suspension of the microspheres include aqueous sodium chloride at 0.9 % concentration by weight, polyvinylpyrrolidone (PVP), sold under the trade designation Plasdone K-30 and Povidone by GAF Corp, contrast media sold under the trade designation Visipaque or Omnipaque by Amersham Biosciences of Uppsala, Sweden, contrast media sold under the trade designation Optiray by Mallinckrodt, Inc, of St. Louis, Missouri, a contrast media sold under the trade designation Metrizamide by Nyegard & Co. of Oslo, Norway, a contrast media sold under the trade designation Renografin 76 by E. R. Squibb & Co., 50% dextrose solutions and saline.

The radiolabeled microspheres may also be administered to the patient in combination with agents that enhance the efficacy of radiotherapy, so-called radiosensitizers. Without being bound by theory, radiosensitizers are believed to enhance the therapeutic effect of radiation by either amplifying the damage to cells by the radiotherapy, or by inhibiting radiation-damaged cells from multiplying or repairing themselves. Examples of radiosensitizers are gemcitabine, docetaxel, and nitrated imidazoles, such as metronidazole and nimorazole.

### Certain Methods for Producing ³²P-Containing Radioactive Microspheres for Endovascular Therapeutic Intervention

Therapeutic embolization is the deliberate occlusion of vascular structures using a variety of agents. Recanalization is a common phenomenon that decreases the efficacy of such embolization procedures. It is routinely observed after coil occlusion of arteries or aneurysms, but it has also been described for other embolic agents, including particles. Raymond J. et al. In situ beta radiation to prevent recanalization after coil embolization of cerebral aneurysms. Stroke, 2002 Feb;33(2):421-427; Raymond J. et al. Beta radiation and inhibition of recanalization after coil embolization of canine arteries and experimental aneurysms: how should radiation be delivered? Stroke, 2003 May;34(5):1262-1268; Hall WA et al. Recanalization of spinal arteriovenous malformations following embolization. J Neurosurg. 1989 May;70(5):714-20; and Sorimachi T. et al. Embolization of cerebral arteriovenous malformations achieved with polyvinyl alcohol particles: angiographic reappearance and complications. AJNR Am J Neuroradiol. 1999 Aug;20(7):1323-8.

Embolization of arteriovenous malformations or dural fistulae with particles, even when they are made of unresorbable material, such as polyvinyl alcohol, is commonly followed by recanalization and recurrences. Davidson GS, Terbrugge KG. Histologic long-term follow-up after embolization with polyvinyl alcohol particles. AJNR Am J Neuroradiol. 1995 Apr;16(4 Suppl):843-846. Particles are relatively safe, easy to use, flow-guided embolic agents that are helpful when a proximal occlusion is insufficient and when the goal of the procedure necessitates the obliteration of a normal or pathologic vascular bed. Particles can be manufactured in a wide range of sizes. They usually carry lesser risks of ischemic complications than liquid agents. Because of frequent recanalization, particles are no longer recommended unless the goal is short-term or preoperative devascularization.

Recanalization is a cellular process that can reliably be inhibited by radiation. Raymond J. et al. Recanalization of arterial thrombus, and inhibition with beta-radiation in a new murine carotid occlusion model: MRNA expression of angiopoietins, metalloproteinases, and their inhibitors. J Vasc Surg. 2004 Dec;40(6):1190-8. Hydrogel microspheres are frequently used for tumor devascularization, again as a preoperative procedure, or as a non-invasive management of benign tumors, such as uterine fibroids. Spies JB et al. Initial experience with use of tris-acryl gelatin microspheres for uterine artery embolization for leiomyomata. J Vasc Interv Radiol 2001;12:1059-1063; and Pelage JP, Le Dref O, Beregi JP, Nonent M, Robert Y, Cosson M, Jacob D, True JB, Laurent A, Rymer R. Limited Uterine Artery Embolization with Tris-acryl Gelatin Microspheres for Uterine Fibroids. J Vase Interv Radiol 2003;14:15-20. The addition of a beta-emitting isotope to hydrogel microspheres could decrease recanalization while preserving the advantages of particle embolization.

The internal delivery of radioactivity, compared to external delivery by radioactive beams, allows the use of less penetrating radioactive sources and by definition healthy tissues do not have to be traversed to reach the target. Hence embolization of the tumoral vascular bed with radioactive microspheres allows delivery of a large radiation dose to the tumor, while minimizing radiation damage to surrounding tissues. In situ radiation can also palliate recanalization, a drawback associated with particle embolization; Thus, radioactive microspheres are promising for the treatment of vascular disorders, such as cerebral arteriovenous malformations or dural fistulae.

Useful isotopes for internal radiotherapy usually emit beta particles or soft x-rays. Physical properties of certain isotopes used in this study are summarized in Table 2. The effective penetration range in tissues depends on the nature of radiation: it is up to about 90 µm (10 cell layers) for α, a few but never more than 12 mm for β-emitters, and up to several centimeters for γ-emitters. Häfeli UO Radioactive microspheres for medical applications. In: Bulte J, de Kuyper M (eds) Focus on biotechnology. Kluwer Academic Publishing, 213-248 (2001). Pure β-emitters, such as ³²P have been favored during the last decade, because the presence of high energy γ-rays in other radioisotopes led to higher than necessary radiation doses to non-targeted organs and hospital personnel. However, a certain amount of low energy γ-radiation (as in ¹⁹⁸Au) can actually be useful for imaging, either during or after application of the radioactive microspheres. The radiologist may be able to adjust the necessary amounts of radioactivity during implantation with the help of a γ-camera or detector.

**Table 2**

| Nuclear Properties of ³²P and ¹⁹⁸Au | | |
|---|---|---|
| Property | ³²P | ¹⁹⁸Au |
| Half life | 14.3 days | 2.7 days |
| Maximum beta energy | 1710.2 keV | 960.7 keV |
| Maximum tissue penetration | 7.9 mm | 3.9 mm |
| Gamma emissions for imaging | none | 411.8 keV (95.5%) |
| Thermal neutron cross section | 0.19 barn | 99 barns |

There are two archetypal approaches to producing radioactive microspheres. The first approach consists of incorporation of an element into a material and then radioactive transmutation through neutron bombardment. A clinically available example is ⁹⁰Yttrium-containing glass spheres (Theraspheres, MDS, Ottawa, Canada), in which ⁸⁹Yttrium oxide is transmuted to radioactive ⁹⁰Yttrium through neutron bombardment. Wollner I et al. Effects of hepatic arterial yttrium 90 glass microspheres in dogs. Cancer. 1988 Apr 1;61(7):1336-1344.

Another approach is to bind radioactive elements to preformed microspheres. For example, ⁹⁰Yttrium can be bound to an Aminex resin and then precipitated inside beads by washing with phosphate solutions, leading to insoluble ⁹⁰Yttrium phosphate (Sirtex Medical Limited, Australia). Gray BN, US Patent Application US 2003/0007928. Another example is Technetium-coated albumin microspheres in which the Technetium is precipitated on top of the albumin. Rhodes BA et al. Radioactive albumin microspheres for studies of pulmonary circulation. Radiology. 1969 Jun;92(7):1453-1460. Two other approaches to producing radioactive microspheres are radiolabeling during microsphere preparation, and in situ neutron-capture therapy using non-radioactive microspheres. Most approaches currently available are expensive and the embolic material is poorly adapted to embolization procedures.

Remarkably, we have developed approaches for transforming hydrogel beads into radioactive beads. The radioactive beads were then tested in animal models commonly used in the assessment of embolic agents. Massoud TF et al. An experimental arteriovenous malformation model in swine: anatomic basis and construction technique. AJNR Am J Neuroradiol. 1994 Sep;15(8):1537-45; Siekmann R et al. Modification of a previously described arteriovenous malformation model in the swine: endovascular and combined surgical/endovascular construction and hemodynamics.AJNR Am J Neuroradiol. 2000 Oct;21(9):1722-5; Raymond J et al. Temporary vascular occlusion with poloxamer 407. Biomaterials. 2004 Aug;25(18):3983-9; and Larsen NE et al. Hylan gel composition for percutaneous embolization. J Biomed Mater Res. 1991 Jun;25(6):699-710.

The present invention exploits the binding properties of zirconium and ³²P phosphate, a commonly used pure β-emitter. During β-decay, a neutron in the unstable nucleus is transformed into a proton, an electron and a neutrino. Additionally, free energy is produced and released in the form of kinetic energy given to the electron and the neutrino. Passing through tissue, the ejected .beta.-particles interact with other atoms and lose energy, leading to excited and ionized atoms. These activated species (e.g., free radicals) are responsible for therapeutic effects, but also toxicity. Zirconium beads are used in liquid chromatography to attract phosphate. Schafer W A, Carr P W. Chromatographic characterization of a phosphate-modified zirconia support for bio-chromatographic applications. J Chromatogr. Dec. 20, 1991;587(2):149-60. This property was directly applied to microspheres for embolization. Zirconium-containing hydrogel beads were loaded with substantial amounts of ³²P without any difficulty. After the initial washout of poorly bound or contaminating ³²P, subsequent washes showed relatively tight linkage of the isotope to the microspheres, with 11% of in vitro leaching. Such a simple method circumvents the problem of storage and distribution of a radioactive inventory of microspheres with a limited half-life (14 days for ³²P), since microspheres may be prepared on site, in the radiopharmacy for example.

### Embolization in Conjunction with Drug Delivery

New ways of delivering drugs at the right time, in a controlled manner, with minimal side effects, and greater efficacy per dose are sought by the drug-delivery and pharmaceutical industries. The polymers used in the embolization methods provided have physico-chemical characteristics that make them suitable delivery vehicles for conventional small-molecule drugs, as well as new macromolecular drugs (*e.g.,* peptides) or other therapeutic products. The polymers may be used for drug delivery in either their radiolabelled or non-radiolabelled form. Labeling the drug-loaded polymers with an imageable radioisotope, such as indium-111, will aid in determining the drug dose to the targeted tissue. Labeling the drug-loaded polymer with a therapeutic radioisotope, such as phosphorous-32 (as phosphate), may provide a synergistic effect with the drug therapy. A pharmaceutic effect is one which seeks to treat the source or symptom of a disease or physical disorder. Pharmaceutics include those products subject to regulation under the FDA pharmaceutic guidelines, as well as consumer products. Importantly, the compositions used in the embolization methods provided are capable of solubilizing and releasing bioactive materials. Release of the drug would occur through diffusion or network erosion mechanisms.

Those skilled in the art will appreciate that the compositions used in the embolization methods provided may be used in a wide variety of pharmaceutic and personal care applications. To prepare a pharmaceutic composition, an effective amount of pharmaceutically active agent(s) which imparts the desirable pharmaceutic effect is incorporated into the gelling composition used in the embolization methods of the invention. Preferably, the selected agent is water soluble, which will readily lend itself to a homogeneous dispersion throughout the gelling composition. For materials which are not water soluble, it is also within the scope of the embolization methods provided to disperse or suspend lipophilic material throughout the composition. Myriad bioactive materials may be delivered using the methods provided; the delivered bioactive material includes anesthetics, antimicrobial agents (antibacterial, antifungal, antiviral), anti-inflammatory agents, diagnostic agents, and wound-healing agents.

Because the compositions used in the methods provided are suited for application under a variety of physiological conditions, a wide variety of pharmaceutically active agents may be incorporated into and administered from the composition. The pharmaceutic agent loaded into the polymer networks of the polymer may be any substance having biological activity, including proteins, polypeptides, polynucleotides, nucleoproteins, polysaccharides, glycoproteins, lipoproteins, and synthetic and biologically engineered analogs thereof.

A vast number of therapeutic agents may be incorporated in the polymers used in the methods provided. In general, therapeutic agents which may be administered via the methods include, without limitation: antiinfectives such as antibiotics and antiviral agents; analgesics and analgesic combinations; anorexics; antihelmintics; antiarthritics; antiasthmatic agents; anticonvulsants; antidepressants; antidiuretic agents; antidiarrheals; antihistamines; antiinflammatory agents; antimigraine preparations; antinauseants; antineoplastics; antiparkinsonism drugs; antipruritics; antipsychotics; antipyretics, antispasmodics; anticholinergics; sympathomimetics; xanthine derivatives; cardiovascular preparations including calcium channel blockers and beta-blockers such as pindolol and antiarrhythmics; antihypertensives; diuretics; vasodilators including general coronary, peripheral and cerebral; central nervous system stimulants; cough and cold preparations, including decongestants; hormones such as estradiol and other steroids, including corticosteroids; hypnotics; immunosuppressives; muscle relaxants; parasympatholytics; psychostimulants; sedatives; and tranquilizers; and naturally derived or genetically engineered proteins, polysaccharides, glycoproteins, or lipoproteins. Suitable pharmaceuticals for parenteral administration are well known as is exemplified by the Handbook on Injectable Drugs, 6th edition, by Lawrence A. Trissel, American Society of Hospital Pharmacists, Bethesda, Md., 1990.

The pharmaceutically active compound may be any substance having biological activity, including proteins, polypeptides, polynucleotides, nucleoproteins, polysaccharides, glycoproteins, lipoproteins, and synthetic and biologically engineered analogs thereof. The term "protein" is art-recognized and for purposes of this invention also encompasses peptides. The proteins or peptides may be any biologically active protein or peptide, naturally occurring or synthetic.

Examples of proteins include antibodies, enzymes, growth hormone and growth hormone-releasing hormone, gonadotropin-releasing hormone, and its agonist and antagonist analogues, somatostatin and its analogues, gonadotropins such as luteinizing hormone and follicle-stimulating hormone, peptide T, thyrocalcitonin, parathyroid hormone, glucagon, vasopressin, oxytocin, angiotensin I and II, bradykinin, kallidin, adrenocorticotropic hormone, thyroid stimulating hormone, insulin, glucagon and the numerous analogues and congeners of the foregoing molecules. The pharmaceutical agents may be selected from insulin, antigens selected from the group consisting of MMR (mumps, measles and rubella) vaccine, typhoid vaccine, hepatitis A vaccine, hepatitis B vaccine, herpes simplex virus, bacterial toxoids, cholera toxin B-subunit, influenza vaccine virus, bordetela pertussis virus, vaccinia virus, adenovirus, canary pox, polio vaccine virus, plasmodium falciparum, bacillus calmette geurin (BCG), klebsiella pneumoniae, HIV envelop glycoproteins and cytokins and other agents selected from the group consisting of bovine somatropine (sometimes referred to as BST), estrogens, androgens, insulin growth factors (sometimes referred to as IGF), interleukin I, interleukin II and cytokins. Three such cytokins are interferon-beta, interferon-gamma and tuftsin.

Examples of bacterial toxoids that may be incorporated in the compositions used in the embolization methods provided are tetanus, diphtheria, pseudomonas A, mycobaeterium tuberculosis. Examples of that may be incorporated in the compositions used in the embolization methods are HIV envelope glycoproteins, *e.g.,* gp 120 or gp 160, for AIDS vaccines. Examples of anti-ulcer H2 receptor antagonists that may be included are ranitidine, cimetidine and famotidine, and other anti-ulcer drugs are omparazide, cesupride and misoprostol. An example of a hypoglycaemic agent is glizipide.

Classes of pharmaceutically active compounds which can be loaded into that may be incorporated in the compositions used in the embolization methods include, but are not limited to, anti-AIDS substances, anti-cancer substances, antibiotics, immunosuppressants (*e.g.,* cyclosporine) anti-viral substances, enzyme inhibitors, neurotoxins, opioids, hypnotics, antihistamines, lubricants tranquilizers, anti-convulsants, muscle relaxants and anti-Parkinson substances, anti-spasmodics and muscle contractants, miotics and anti-cholinergics, anti-glaucoma compounds, anti-parasite and/or anti-protozoal compounds, anti-hypertensives, analgesics, anti-pyretics and anti-inflammatory agents such as NSAIDs, local anesthetics, ophthalmics, prostaglandins, anti-depressants, anti-psychotic substances, anti-emetics, imaging agents, specific targeting agents, neurotransmitters, proteins, cell response modifiers, and vaccines.

Exemplary pharmaceutical agents considered to be particularly suitable for incorporation in the compositions used in the embolization methods include but are not limited to imidizoles, such as miconazole, econazole, terconazole, saperconazole, itraconazole, metronidazole, fluconazole, ketoconazole, and clotrimazole, luteinizing-hormone-releasing hormone (LHRH) and its analogues, nonoxynol-9, a GnRH agonist or antagonist, natural or synthetic progestrin, such as selected progesterone, 17-hydroxyprogeterone derivatives such as medroxyprogesterone acetate, and 19-nortestosterone analogues such as norethindrone, natural or synthetic estrogens, conjugated estrogens, estradiol, estropipate, and ethinyl estradiol, bisphosphonates including etidronate, alendronate, tiludronate, resedronate, clodronate, and pamidronate, calcitonin, parathyroid hormones, carbonic anhydrase inhibitor such as felbamate and dorzolamide, a mast cell stabilizer such as xesterbergsterol-A, lodoxamine, and cromolyn, a prostaglandin inhibitor such as diclofenac and ketorolac, a steroid such as prednisolone, dexamethasone, fluromethylone, rimexolone, and lotepednol, an antihistamine such as antazoline, pheniramine, and histiminase, pilocarpine nitrate, a beta-blocker such as levobunolol and timolol maleate. As will be understood by those skilled in the art, two or more pharmaceutical agents may be combined for specific effects. The necessary amounts of active ingredient can be determined by simple experimentation.

By way of example only, any of a number of antibiotics and antimicrobials may be included in the polymers used in the methods. Antimicrobial drugs preferred for inclusion in compositions used in the embolization methods of the invention include salts of lactam drugs, quinolone drugs, ciprofloxacin, norfloxacin, tetracycline, erythromycin, amikacin, triclosan, doxycycline, capreomycin, chlorhexidine, chlortetracycline, oxytetracycline, clindamycin, ethambutol, hexamidine isethionate, metronidazole, pentamidine, gentamicin, kanamycin, lineomycin, methacycline, methenamine, minocycline, neomycin, netilmicin, paromomycin, streptomycin, tobramycin, miconazole and amanfadine and the like.

By way of example only, in the case of anti-inflammation, non-steroidal anti-inflammatory agents (NSAIDS) may be incorporated in the compositions used in the embolization methods, such as propionic acid derivatives, acetic acid, fenamic acid derivatives, biphenylcarboxylic acid derivatives, oxicams, including but not limited to aspirin, acetaminophen, ibuprofen, naproxen, benoxaprofen, flurbiprofen, fenbufen, ketoprofen, indoprofen, pirprofen, carporfen, and bucloxic acid and the like.

### Embolization Kits

The methods may also be practiced using an embolization kit. Such kits may contain a metal-labeled microsphere in sterile form, and may include a sterile container of an acceptable reconstitution liquid. Suitable reconstitution liquids are disclosed in Remington's Pharmaceutical Sciences and The United States Pharmacopia-- The National Formulary. Such kits may also include, if desired, other conventional kit components, such as, for example, one or more carriers, one or more additional vials for mixing. Instructions, either as inserts or labels, indicating quantities of the embolic composition and carrier, guidelines for mixing these components, and protocols for administration may also be included in the kit. Sterilization of the containers and any materials included in the kit and lyophilization (also referred to as freeze-drying) of the embolic composition may be carried out using conventional sterilization and lyophilization methodologies known to those skilled in the art.

Lyophilization aids useful in the embolization kits include but are not limited to mannitol, lactose, sorbitol, dextran, Ficoll, and polyvinylpyrrolidine (PVP). Stabilization aids useful in the embolization kits include but are not limited to ascorbic acid, cysteine, monothioglycerol, sodium bisulfite, sodium metabisulfite, gentisic acid, and inositol. Bacteriostats useful in the embolization kits include but are not limited to benzyl alcohol, benzalkonium chloride, chlorobutanol, and methyl, propyl or butyl paraben. A component in an embolization kit can also serve more than one function. A reducing agent can also serve as a stabilization aid, a buffer can also serve as a transfer ligand, a lyophilization aid can also serve as a transfer, ancillary or co-ligand and so forth.

The absolute and relative amounts of each component of an embolization kit are determined by a variety of considerations that are in some cases specific for that component and in other cases dependent on the amount of another component or the presence and amount of an optional component. In general, the minimal amount of each component is used that will give the desired effect of the formulation. The desired effect of the formulation is that the end-user of the embolization kit may practice the embolization methods with a high degree of certainty that the subject will not be harmed.

The embolization kits also contain written instructions for the practicing end-user. These instructions may be affixed to one or more of the vials or to the container in which the vial or vials are packaged for shipping or may be a separate insert, termed the package insert.

### Selected Clinical Applications of Radionuclide Microspheres

As discussed above, embolization typically is performed using angiographic techniques with guidance and monitoring, *e.g.,* fluoroscopic or X-ray guidance, to deliver an embolizing agent to vessels or arteries. Further, a vasodilator (*e.g.,* adenosine) may be administered to the patient beforehand, simultaneously, or subsequently, to facilitate the procedure.

Importantly, while portions of the subsequent description include language relating to specific clinical applications of embolization, all types of embolization processes are considered to be within the contemplation of the methods provided. Specifically, one of skill in the medical or embolizing art will understand and appreciate how microparticles of hydrogels as described herein can be used in various embolization processes by guiding a delivery mechanism to a desired vascular body site, and delivering an amount of the microparticles to the site, to cause restriction, occlusion, filling, or plugging of one or more desired vessels and reduction or stoppage of blood flow through the vessels. Factors that might be considered, controlled, or adjusted for, in applying the process to any particular embolization process might include the chosen composition of the microparticles (*e.g.,* to account for imaging, tracking, and detection of a radiopaque particle substrate); the amount of microparticles delivered to the body site; the method of delivery, including the particular equipment (*e.g.,* catheter) used and the method and route used to place the dispensing end of the catheter at the desired body site, etc. Each of these factors will be appreciated by one of ordinary skill, and can be readily dealt with to apply the described methods to innumerable embolization processes.

### A. Head and Neck Disorders

In the head and neck, embolotherapy most often is performed for epistaxis and traumatic hemorrhage. Otorhinolaryngologists differentiate anterior and posterior epistaxis on anatomic and clinical bases. Epistaxis results from a number of causes, including environmental factors such as temperature and humidity, infection, allergies, trauma, tumors, and chemical irritants. An advantage of embolization over surgical ligation is the more selective blockade of smaller branches. By embolizing just the bleeding branch, normal blood flow to the remainder of the internal maxillary distribution is retained. Complications of embolization may include the reflux of embolization material outside the intended area of embolization, which, in the worst case, may result in stroke or blindness. Embolization has been proven more effective than arterial ligation. Although embolization has a higher rate of minor complications, no difference in the rate of major complications was found. For traumatic hemorrhage, the technique of embolization is the same as for epistaxis. Because of the size of the arteries in the head and neck, microcatheters are often required.

### B. Thorax Disorders

In the thorax, the two main indications for embolization in relation to hemorrhage are: (1) pulmonary arteriovenous malformations (PAVM); and (2) hemoptysis. PAVMs usually are congenital lesions, although they may occur after surgery or trauma. The congenital form is typically associated with hereditary hemorrhagic telangiectasia, also termed Rendu-Osler-Weber syndrome. There is a genetic predisposition to this condition. PAVMs can be single or multiple, and if large enough, can result in a physiologic right-to-left cardiac shunt. Clinical manifestations of the shunt include cyanosis and polycythemia. Stroke and brain abscesses can result from paradoxical embolism. PAVMs also may hemorrhage, which results in hemoptysis.

Treatment options for PAVMs include surgery and transcatheter therapy. The treatment objective is to relieve the symptoms of dyspnea and fatigue associated with the right-to-left shunt. In addition, if the patient suffers from paradoxical embolism, treatment prevents further episodes. As a result of the less invasive nature of the procedure and excellent technical success rate, embolization currently is considered the treatment of choice for PAVM, whether single or multiple. Embolotherapy is the clear treatment of choice for PAVMs.

Bronchial artery embolization is performed in patients with massive hemoptysis, defined as 500 cm³ of hemoptysis within a 24-hour period. Etiologies vary and include bronchiectasis, cystic fibrosis, neoplasm, sarcoidosis, tuberculosis, and other infections. Untreated, massive hemoptysis carries a high mortality rate. Death most often results from asphyxiation rather than exsanguination. Medical and surgical treatments for massive hemoptysis usually are ineffective, with mortality rates ranging from 35-100%. Embolization has an initial success rate of 95%, with less morbidity and mortality than surgical resection. Consequently, transcatheter embolization has become the therapy of choice for massive hemoptysis, with surgical resection currently reserved for failed embolization or for recurrent massive hemoptysis following multiple prior embolizations.

### C. Abdomen and Pelvis Disorders

Many indications for embolization in the abdomen and pelvis exist. For embolization of hemorrhage, the most common indication is acute GI hemorrhage. Solid organ injury, usually to the liver and spleen, can readily be treated with embolization. Other indications exist, such as gynecologic/obstetric-related hemorrhage and pelvic ring fractures.

Once the source of bleeding is identified, an appropriate embolization procedure can be planned. The technique for embolization is different for upper GI bleeding and lower GI bleeding. The vascular supply in the UGI tract is so richly collateralized that relatively nonselective embolizations can be performed without risk of infarcting the underlying organs. Conversely, the LGI tract has less collateral supply, which necessitates more selective embolizations.

Outside the GI tract, there are organ specific considerations when performing embolizations in the abdomen. For instance, the liver has a dual blood supply, with 75% of the total supply from the portal vein and 25% from the hepatic artery. The hepatic artery invariably is responsible for hemorrhage resulting from trauma due to its higher blood pressure compared to the portal vein. Therefore, all embolizations in the liver are performed in the hepatic artery and not in the portal vein. Because of the dual blood supply, occlusion of large branches of the hepatic artery can be performed without risk of necrosis.

In contrast, embolizations of the spleen always should be performed as distally as possible. Occlusion of the splenic artery can result in splenic necrosis and the possibility of a splenic abscess postembolization. If occlusion of the entire splenic artery is contemplated for traumatic hemorrhage, total splenectomy instead of embolization or total splenectomy postembolization should be performed.

Further indications for hemorrhage embolization in the abdomen and pelvis include postpartum, postcesarean, and postoperative bleeding. Differential diagnoses for postpartum bleeding include laceration of the vaginal wall, abnormal placentation, retained products of conception, and uterine rupture. Conservative measures for treating postpartum bleeding include vaginal packing, dilatation and curettage to remove retained products, IV and intramuscular medications (e.g., oxytocin, prostaglandins), and uterine massage. When conservative methods fail, embolization is a safe and effective procedure for controlling pelvic hemorrhage, avoids surgical risks, preserves fertility, and shortens hospital stays.

Finally, embolization of the internal iliac arteries is valuable in patients with hemodynamically unstable pelvic fractures. Protocols for trauma include treatment of associated soft-tissue injury first, followed by stabilization of the pelvic ring. Patients with persistent hemodynamic instability are candidates for embolization. As in other clinical settings, angiography is used to identify the source of hemorrhage, and a selective embolization is performed.

### D. Cancer

Given the increased skills of interventional radiologists, there is increasing interest in selective radionuclide therapy. Many kinds of radiolabeled particles and radionuclides have been tested for local treatment of a variety of tumors in organs, including liver, lung, tongue, spleen and soft tissue of extremities. The purpose of this treatment is the superselective application of suitable radioactive (high energetic β-emitters) particles to deliver high doses to the tumor, with as little surrounding tissue damage as possible. These new treatment methods are promising particularly for cancers with a poor prognosis and without other adequate therapies, such as primary and metastatic malignancies of the liver.

Patients with primary or metastatic tumors were treated by radio-embolization via a catheter or direct injection of beads into the tumor with a needle (Int. J. Radiation Oncology Biol. Phys. 1990, 18, 619; J. Nucl. Med. 1996, 37, 958). Most studies describe administration of microspheres to patients via a catheter, whereby the tip was placed in the hepatic artery. The spheres eventually lodge in the microvasculature of the liver and tumor, remaining until the complete decay of the radioisotope. Lung shunting and tumor-to-normal liver ratio was determined after infusion of ^{99m}Tc-labeled macro aggregated albumin, and microspheres were subsequently administered to patients (Brit. J. Rad. 1997, 70, 823). Tumor-to-normal liver ratio was approximately 3-5 (Clin. Cancer Res. 1999, 5, 3024s). In some studies the blood flow within the liver was temporarily redirected in favour of the tumor by a bolus infusion of a vasoconstrictor, and the spheres were then embolized into the arterial circulation. While external beam radiation causes radiation hepatitis at doses above 30-35 Gy the liver can tolerate up to 80-150 Gy, using internal radionuclide therapy (Am. J. Roentgenol. Radium Ther. Nucl. Med. 1965, 93, 200). Increased longevity, pain relief, tumor response and total clinical improvement are frequently reported.

Chemo-embolization with ethylcellulose microspheres of 100-450 µm has been used in the treatment of maxillary tumors. The role of intra-arterial radioisotope therapy in the treatment of head and neck cancer is just beginning in rabbits, in the work of van Es et al. (Lab. Anim. 1999, 33, 175). The optimal size of microspheres for treatment of unresectable head-and-neck cancer is still to be established. Some embolizations in the treatment of head-and-neck cancer have been carried out with particles of 100-450 pm (Radiation Med. 1998, 16, 157).

Intra-arterial administration of ⁹⁰Y-microspheres has been carried out in the spleen (Cancer 1972, 31, 90). Of nine patients with lymphosarcoma, five manifested no clinical response after splenic irradiation. One patient who complained of weakness, rapid fatigue and anorexia, had relief of all symptoms after splenic irradiation.

### E. Radioactive Synovectomy in Treatment of Arthritis

Current medical management of rheumatoid arthritis includes patient education, appropriate rest and physical therapy, and the use of anti- inflammatory drugs for relief of pain and inflammation (The Management of Rheumatoid Arthritis. Textbook of Rheumatology, 2nd ed., W. B. Saunders Co. Philadelphia, 1985, p. 979). Patients who do not respond to these modalities may require therapy with anti-malarial agents, such as hydroxychloroquine (American Journal of Medicine, 1983, 75, 46), or remission-inducing agents including gold salts (Ann. Rheum. Dis. 1961, 20, 315), penicillamine (Lancet 1973, 1, 275), or azathioprine (Arthritis Rheum. 1978, 21, 539). Despite the efficacy of these drugs, patient response is variable and improvement may not occur until treatment has extended for three to six months. When a few joints remain swollen and painful and interfere with the patient's progress, intra-articular instillation of corticosteroids may be used as an adjunct to systemic therapy. This local remedy, however, may be ineffective or may last only a few days (Textbook of Rheumatology, supra, p 546).

Surgery may be used in several different ways to help a patient with rheumatoid arthritis. Surgery can help relieve pain, it can prevent further deformity and loss of function, or at least allay these problems, and when destruction has occurred, reconstructive procedures can return function to a part or a limb (Textbook of Rheumatology, supra, p. 1787).

Most of the operations done on rheumatoid patients relieve pain. Fusions of joints, total joint replacement and synovectomy are examples of procedures that significantly reduce pain. Conaty (Journal of Bone and Joint Surgery, 1973, 55(A), 301) states that in rheumatoid arthritis, synovectomy was the most successful procedure for preserving motion of a joint, except for total joint arthroplasty. This procedure, then, is preventive. Even so, eventually the synovium regenerates and the process continues (Journal of Bone and Joint Surgery, 1973, 55(A), 287). Total joint surgery will relieve any or all of the aforementioned disabilities, but brings with it other problems that must be taken into consideration by the surgeon. Some of these are: 1) cost, 2) the risk of infection, 3) the fact that the implants may come loose and be painful, and 4) the fact that the implant may break with unusual use.

Chemical and radioisotope synovectomy (synoviorthesis) constitutes an effective alternative to operative therapy. The advantages of synoviorthesis are: 1) simple techniques employed in their use, 2) decreased or no hospitalization, 3) lower costs, 4) early and easier mobilization of the patient, and 5) a surgical synovectomy remains an alternative treatment should the synoviorthesis not work.

In general, the results of radioisotope synoviorthesis appear to be superior to those attained with chemical synovectomy. (Rev. Rhum. 1973, 40, 255; Acta Rheum. Scand. 1970, 16, 271; Rev. Rhum. 1973, 40, 205). Radioactive substances used include gold-198, yttrium-90 citrate, yttrium-90 resin, rhenium-186, erbium-169, yttrium-90 ferric metal hydroxide, radium-224 and phosphorus-32 chromic phosphate.

Treatment of the different depths of diseased synovium in joints of disparate size, such as the finger joints and the knee, requires isotopes of different average beta range. It is important to achieve a "kill" of sufficient depth to be efficacious without causing significant necrosis of overlying normal tissues.

Sledge et al. (Arthritis and Rheumatism, 1986, 29, 153) have used macroaggregates of ferric metal hydroxide (FHMA) combined with dysprosium-165. This compound does present the problem of some leakage to local lymph nodes and other tissues. Also, dysprosium-165 has a half-life of 2.3 hours, making it necessary for the patient to be close to a nuclear reactor, severely limiting the use of this radioisotope. Even with these drawbacks, the clinical results were noteworthy, as 80% of patients treated for chronic synovitis of the knee with dysprosium-165-FHMA were improved at one year, and nearly 90% of patients with stage 1 roentgenographic changes had excellent, good, or fair results (Clinical Orthopaedics and Related Research 1984, 182, 37). These results and the results of others (European Journal of Nuclear Medicine 1985, 10, 446; Ann. Rheum. Dis. 1984, 43, 620; Annals of the Rheumatic Diseases 1983, 42, 132; Use of Radiocolloids for Intra-Articular Therapy for Synovitis, In Therapy in Nuclear Medicine, Grune and Stratton, Inc., New York, 1978, p. 147; European Journal of Nuclear Medicine 1985, 10, 441) show that radiation synoviorthesis has a role in the treatment of inflammatory synovitis.

Some conventional microspheres might not be suitable for use in radiation synovectomy by reason of the radionuclides incorporated therein having relatively long physical half-lives. Therefore, there is a continuing need, therefore, for improved microspheres and methods for radiation synovectomy of arthritic joints.

### F. Radioactive Synovectomy in Haemophilia Patients

The indication for a synoviorthesis (medical synovectomy) is chronic haemophilic synovitis causing recurrent haemarthroses that are unresponsive to haematological treatment. Synoviorthesis is the intra-articular injection of a certain material to diminish the degree of synovial hypertrophy, decreasing the number and frequency of haemarthroses. There are two basic types of synoviorthesis: chemical synoviorthesis and radiation synoviorthesis. On average, the efficacy of the procedure ranges from 76 to 80%, and can be performed at any age. The procedure slows the cartilaginous damage which intra-articular blood tends to produce in the long term. Synoviorthesis can be repeated up to three times with 3-month intervals if radioactive materials are used (Yttrium-90 and Phosphorus-32), or weekly up to 10-15 times if rifampicin (chemical synovectomy) is used. After 30 years of using radiation synovectomy worldwide, no damage has been reported in relation to the radioactive materials. Radiation synovectomy is currently the preferred procedure when radioactive materials are available (Haemophilia 2001, 7, 6).

### Definitions

For convenience, certain terms employed in the specification, examples, and appended claims are collected here.

The articles "a" and "an" are used herein to refer to one or to more than one (i.e. to at least one) of the grammatical object of the article. By way of example, "an element" means one element or more than one element.

As used in the present invention, the term "metal" refers to elements which posses metallic character, including the metaloids.

As used in the present invention, the term "radionuclide" refers to a radioactive isotope or element.

Throughout the application, including the claims and figures, references to, e.g., "first radioisotope" and "second radioisotope" serve only to establish separate identities for the radioisotopes in question; in particular, the use of the terms "first" and "second" in this context does not by itself imply anything regarding the order in which the radioisotopes were or will be incorporated into, e.g., a microsphere.

As used in the present invention, the term "biodistribution" refers to the location of the given particle or particles in a biological entity.

As used in the present invention, the term "microsphere" refers to an object that is substantially spherical in shape and has a diameter less than 1 millimeter.

As used in the present invention, the phrase "time of use" refers to the period during which a microsphere is implanted in a patient or subject.

As used in the present invention, the phrase "associated with" means the condition in which two or more substances having any type of physical contact. For example, when a polymeric material is "associated with" metal or metal compound particles, the metal particles may be deposited on the surface of the polymeric material, within the material, or, if the material is porous, within the pores of the material, through any type of physical or chemical interactions such as through covalent bond, ionic bond, or van der Waal's bond, or through impregnating, intercalating, or absorbing. According to the present invention, when a polymeric material is associated with metal or metal compound particles, it is "labeled" with the metal or metal compound particles.

As used in the present invention, the term "implant" means a substance that is placed or embedded at least in part within the tissue of a mammal. An "implantable" substance is capable of being placed or embedded within the tissue through whatever means. For example, within the meaning of the present invention, a piece of traditional prosthetic device is an implant. So are substances, such as microparticles, that are placed within the dermal tissue of a mammal.

As used in the present invention, the tenn "embolization" means the occlusion or blockage of a blood vessel. The occlusion or blockage may occur either due to blood clots or emboli as a result of a physiological condition or due to an artificial act of embolic materials. In this regard, according to the present invention, an embolus is different from an implant.

As used herein, the term "polymer" means a molecule, formed by the chemical union of two or more oligomer units. The chemical units are normally linked together by covalent linkages. The two or more combining units in a polymer can be all the same, in which case the polymer is referred to as a homopolymer. They can be also be different and, thus, the polymer will be a combination of the different units. These polymers are referred to as copolymers.

As used in the present invention, the term "hydrogel" refers to a polymeric composition, comprising at least 50% water by weight, and can comprise a wide variety of polymeric compositions and pore structures.

The term "contrast-enhancing" refers to materials capable of being monitored during injection into a mammalian subject by methods for monitoring and detecting such materials, for example by radiography or fluoroscopy. An example of a contrast-enhancing agent is a radiopaque material. Contrast-enhancing agents including radiopaque materials may be either water soluble or water insoluble. Examples of water soluble radiopaque materials include metrizamide, iopamidol, iothalamate sodium, iodomide sodium, and meglumine. Examples of water insoluble radiopaque materials include metals and metal oxides such as gold, titanium, silver, stainless steel, oxides thereof, aluminum oxide, zirconium oxide, etc.

As used in the present invention, the term "injectable" means capable of being administered, delivered or carried into the body via a needle, a catheter, or other similar ways.

As used in the present invention, "microparticles" means polymer or combinations of polymers made into bodies of various sizes. The microparticles can be in any shape, although they are often in substantially spherical shape, in which case the microparticles are referred to as "microspheres" or "microbeads."

As used herein, "zirconia" means zirconium dioxide, zirconium metal hydroxide, other hydrated forms of zirconium, and mixtures of any of them. "Zirconia" in this invention may also contain zirconium acetate, from which it may be derived.

For purposes of this invention, the chemical elements are identified in accordance with the Periodic Table of the Elements, CAS version, Handbook of Chemistry and Physics, 67th Ed., 1986-87, inside cover.

### Microspheres of the Invention

One aspect of the present invention relates to a microsphere, according to claim 1.

In certain embodiments, the microsphere further comprising a second radioisotope; wherein the atomic number of the first radioisotope is not the same as the atomic number of the second radioisotope.

In certain embodiments, the present invention relates to the aforementioned microsphere and the attendant definitions, wherein said hydrophilic polymer comprises one or more polymerized monomers selected from the group consisting of acrylics, vinyls, acetals, allyls, cellulosics, methacrylates, polyamides, polycarbonate, polyesters, polyimide, polyolefins, polyphosphates, polyurethanes, silicones, styrenics, and polysaccharides.

In certain embodiments, the present invention relates to the aforementioned microsphere and the attendant definitions, wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide.

In certain reference embodiments, the present invention relates to the aforementioned microsphere and the attendant definitions, wherein said hydrophilic polymer comprises one or more polymerized monomers selected from the group consisting of crosslinked gelatin, N-[tris(hydroxymethyl)methyl]acrylamide and vinylphosphonate.

In certain embodiments, the present invention relates to the aforementioned microsphere and the attendant definitions, wherein said hydrophilic polymer comprises one or more polymerized monomers selected from the group consisting of N-[tris(hydroxymethyl)-methyl]acrylamide and vinylphosphonate.

In certain embodiments, the present invention relates to the aforementioned microsphere and the attendant definitions, wherein said pendant moieties are selected independently from the group consisting of phosphonic acids, phosphates, bisphosphonic acids, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines.

In certain embodiments, the present invention relates to the aforementioned microsphere and the attendant definitions, wherein said pendant moieties are selected independently from the group consisting of phosphonic acids, bisphosphonic acids, sulfonic acids, and carboxylic acids.

In certain embodiments, the present invention relates to the aforementioned microsphere and the attendant definitions, wherein said pendant moieties are phosphonic acids.

In certain embodiments, the present invention relates to the aforementioned microsphere and the attendant definitions, wherein said second radioisotope is technetium-99m, ¹¹¹In or ⁶⁷Ga.

In certain embodiments, the present invention relates to the aforementioned microsphere and the attendant definitions, wherein said second radioisotope is ¹¹¹In.

In certain embodiments, the present invention relates to the aforementioned microsphere and the attendant definitions, wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxy quinolines.

In certain embodiments, the present invention relates to the aforementioned microsphere and the attendant definitions, wherein said hydrophilic polymer comprises one or more polymerized monomers selected from the group consisting of crosslinked gelatin, N-[tris(hydroxymethyl)methyl]acrylamide and vinylphosphonate; and wherein said pendant moieties are selected independently from the group consisting of phosphonic acids, bisphosphonic acids, sulfonic acids, and carboxylic acids.

In certain embodiments, the present invention relates to the aforementioned microsphere and the attendant definitions, wherein said hydrophilic polymer comprises one or more polymerized monomers selected from the group consisting of N-[tris(hydroxymethyl)methyl]-acrylamide and vinylphosphonate; and wherein said pendant moieties are phosphonic acids.

In certain embodiments, the present invention relates to the aforementioned microsphere and the attendant definitions, wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines.

In certain embodiments, the present invention relates to the aforementioned microsphere and the attendant definitions, wherein said hydrophilic polymer comprises one or more polymerized monomers selected from the group consisting of crosslinked gelatin, N-[tris(hydroxymethyl)-methyl]acrylamide and vinylphosphonate; and wherein said pendant moieties are selected independently from the group consisting of phosphonic acids, bisphosphonic acids, sulfonic acids, and carboxylic acids.

In certain embodiments, the present invention relates to the aforementioned microsphere and the attendant definitions, wherein said hydrophilic polymer comprises one or more polymerized monomers selected from the group consisting of N-[tris(hydroxymethyl)methyl]-acrylamide and vinylphosphonate; and wherein said pendant moieties are phosphonic acids.

In certain embodiments, the present invention relates to the aforementioned microsphere and the attendant definitions, wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines.

In certain embodiments, the present invention relates to the aforementioned microsphere and the attendant definitions, wherein said hydrophilic polymer comprises one or more polymerized monomers selected from the group consisting of crosslinked gelatin, N-[tris(hydroxymethyl)methyl]acrylamide and vinylphosphonate; and wherein said pendant moieties are selected independently from the group consisting of phosphonic acids, bisphosphonic acids, sulfonic acids, and carboxylic acids;
and wherein said first radioisotope is in the form of ³²P phosphate.

In certain embodiments, the present invention relates to the aforementioned microsphere and the attendant definitions, wherein said hydrophilic polymer comprises one or more polymerized monomers selected from the group consisting of N-[tris(hydroxymethyl)methyl]-acrylamide and vinylphosphonate; and wherein said pendant moieties are phosphonic acids.

In certain embodiments, the present invention relates to the aforementioned microsphere and the attendant definitions, wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines;
and wherein said second radioisotope is technetium-99m, ¹¹¹In or ⁶⁷Ga.

In certain embodiments, the present invention relates to the aforementioned microsphere and the attendant definitions, wherein said hydrophilic polymer comprises one or more polymerized monomers selected from the group consisting of crosslinked gelatin, N-[tris(hydroxymethyl)-methyl]acrylamide and vinylphosphonate; and wherein said pendant moieties are selected independently from the group consisting of phosphonic acids, bisphosphonic acids, sulfonic acids, and carboxylic acids; and wherein said second radioisotope is technetium-99m, ¹¹¹In or ⁶⁷Ga.

In certain embodiments, the present invention relates to the aforementioned microsphere and the attendant definitions, wherein said hydrophilic polymer comprises one or more polymerized monomers selected from the group consisting of N-[tris(hydroxymethyl)methyl]-acrylamide and vinylphosphonate; and wherein said pendant moieties are phosphonic acids; and wherein said second radioisotope is technetium-99m, ¹¹¹In or ⁶⁷Ga.

In certain embodiments, the present invention relates to the aforementioned microsphere and the attendant definitions, wherein said hydrophilic polymer comprises one or more polymerized monomers selected from the group consisting of N-[tris(hydroxymethyl)methyl]-acrylamide and vinylphosphonate; and wherein said pendant moieties are phosphonic acids; and wherein said second radioisotope is ¹¹¹In. In certain embodiments, the present invention relates to the aforementioned microsphere and the attendant definitions, wherein the ratio of the radioactivity of the second radioisotope to the first radioisotope is in the range from about 1:10 to about 1:10⁷ at the time of use.

In certain embodiments, the present invention relates to the aforementioned microsphere and the attendant definitions, wherein the ratio of the radioactivity of the second radioisotope to the first radioisotope is in the range from about 1:10² to 1:10⁶ at the time of use.

In certain embodiments, the present invention relates to the aforementioned microsphere and the attendant definitions, wherein the ratio of the radioactivity of the second radioisotope to the first radioisotope is in the range from about 1:10⁴ to 1:10⁵ at the time of use.

In certain embodiments, the present invention relates to the aforementioned microsphere and the attendant definitions, wherein the ratio of the radioactivity of the second radioisotope to the first radioisotope is in the range from about 1:10 to 1:10³ at the time of use.

In certain embodiments, the present invention relates to the aforementioned microsphere and the attendant definitions, wherein said first radioisotope is not leached from said microsphere to an extent greater than about 3%; wherein said second radioisotope is not leached from said microsphere to an extent greater than about 3%.

In certain embodiments, the present invention relates to the aforementioned microsphere and the attendant definitions, wherein said first radioisotope is not leached from said microsphere to an extent greater than about 1%; wherein said second radioisotope is not leached from said microsphere to an extent greater than about 1%.

In certain embodiments, the present invention relates to the aforementioned microsphere and the attendant definitions, wherein said microsphere further comprises a biologically active agent.

In certain embodiments, the present invention relates to the aforementioned microsphere and the attendant definitions, wherein said microsphere further comprises a contrast-enhancing agent.

In certain embodiments, the present invention relates to the aforementioned microsphere and the attendant definitions, wherein said contrast-enhancing agent is selected from the group consisting of radiopaque materials, paramagnetic materials, heavy atoms, transition metals, lanthanides, actinides, and dyes.

In certain embodiments, the present invention relates to the aforementioned microsphere and the attendant definitions, wherein the diameter of said microsphere is in the range from about 1-2000 micrometers.

In certain embodiments, the present invention relates to the aforementioned microsphere and the attendant definitions, wherein the diameter of said microsphere is in the range from about 1- 1000 micrometers.

In certain embodiments, the present invention relates to the aforementioned microsphere and the attendant definitions, wherein the diameter of said microsphere is in the range from about 1-500 micrometers.

In certain embodiments, the present invention relates to the aforementioned microsphere and the attendant definitions, wherein the diameter of said microsphere is in the range from about 1-100 micrometers.

In certain embodiments, the present invention relates to the aforementioned microsphere and the attendant definitions, wherein the diameter of said microsphere is in the range from about 10-40 micrometers.

### Methods of the Invention

The present invention also relates to methods of preparing a radioactive microsphere according to claims 12 or 17.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl] -acrylamide, N,N'-methylene-bis-acrylamide, N',N'-diallyl-tartradiamide, and glyoxal-bis-acrylamide; and
converting the zirconium in the microsphere-metal complex to its oxide, polyoxometalate, hydroxide, alkoxide, carboxylate, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and
converting the zirconium in the microsphere-metal complex to its oxide, polyoxometalate, hydroxide, alkoxide, or carboxylate, thereby forming a metal-labeled microsphere; and
combining said metal-labeled microsphere with the first radioisotope being in the form of ³²P phosphate, thereby forming a radioactive metal-labeled microsphere.

Preferably the invention also relates to a method of preparing a radioactive microsphere, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and
converting the zirconium in the microsphere-metal complex to its oxide, polyoxometalate, hydroxide, alkoxide, or carboxylate, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of crosslinked gelatin, N-[tris(hydroxymethyl)-methyl]acrylamide and vinylphosphonate; and
converting the zirconium in the microsphere-metal complex to its oxide, polyoxometalate, hydroxide, alkoxide, or carboxylate, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said pendant moieties are selected independently from the group consisting of phosphonic acids, bisphosphonic acids, sulfonic acids, and carboxylic acids; and
converting the zirconium in the microsphere-metal complex to its oxide, polyoxometalate or hydroxide, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of crosslinked gelatin, N-[tris(hydroxymethyl)-methyl]acrylamide and vinylphosphonate; and wherein said pendant moieties are selected independently from the group consisting of phosphonic acids, bisphosphonic acids, sulfonic acids, and carboxylic acids; and
converting the zirconium in the microsphere-metal complex to its oxide, polyoxometalate, hydroxide, alkoxide, or carboxylate, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of N-[tris(hydroxymethyl)methyl]-acrylamide and vinylphosphonate; and
converting the zirconium in the microsphere-metal complex to its oxide, polyoxometalate, hydroxide, alkoxide, or carboxylate, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said pendant moieties are phosphonic acids; and
converting the zirconium in the microsphere-metal complex to its oxide, polyoxometalate, hydroxide, alkoxide, or carboxylate, thereby forming a metal-labeled microsphere; and
combining said metal-labeled microsphere with the first radioisotope being in the form of ³²P phosphate, thereby forming a radioactive metal-labeled microsphere.

Preferably the invention relates to a method of preparing a radioactive microsphere, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of N-[tris(hydroxymethyl)methyl]-acrylamide and vinylphosphonate; and wherein said pendant moieties are phosphonic acids;
converting the zirconium in the microsphere-metal complex to its oxide, polyoxometalate, hydroxide, alkoxide, or carboxylate, thereby forming a metal-labeled microsphere; and
combining said metal-labeled microsphere with a first radioisotope, wherein said first radioisotope is in the form of ³²P phosphate; thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate or hydroxide or combination thereof and a microsphere comprising a hydrophilic polymer comprising a plurality of pendant moieties, thereby forming a metal-labeled microsphere; wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; wherein said transition-metal, lanthanide or group 13-14 metal oxide, polyoxometalate or metal hydroxide or combination thereof comprises a metal oxide, polyoxometalate or metal hydroxide or combination thereof of zirconium, scandium, yttrium, lanthanum, hafnium, titanium, aluminum, silicon, gallium, indium, thallium, germanium, tin, lead, bismuth, tungsten, tantalum, cerium, praseodymium, neodymium, promethium, samarium, europium, gadolinium, terbium, dysprosium, holmium, erbium, thulium, ytterbium, or lutetium; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; and
combining said metal-labeled microsphere with a first radioisotope, wherein ⁹⁰Y, ³²P, ¹⁸F, ¹⁴⁰La, ¹⁵³Sm, ¹⁶⁵Dy, ¹⁶⁶Ho, ¹⁶⁹Er, ¹⁶⁹Yb, ¹⁷⁷Lu, ¹⁸⁶Re, ¹⁸⁸Re, ¹⁰³Pd, ¹⁹⁸Au, ¹⁹²Ir, ⁹⁰Sr, ¹¹¹In or ⁶⁷Ga; thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; wherein said hydrophilic polymer comprises one or more polymerized monomers selected from the group consisting of N-[tris(hydroxymethyl)methyl]-acrylamide and vinylphosphonate; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; wherein said pendant moieties are phosphonic acids; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 oxide, polyoxometalate, hydroxide, alkoxide, or carboxylate, or combination thereof and a microsphere comprising a hydrophilic polymer comprising a plurality of pendant moieties, thereby forming a metal-labeled microsphere; wherein said transition-metal, lanthanide or group 13-14 metal oxide, polyoxometalate or metal hydroxide or combination thereof is an oxide, polyoxometalate or hydroxide of zirconium or combination thereof; and
combining said metal-labeled microsphere with a first radioisotope, thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; and
combining said metal-labeled microsphere with a first radioisotope, wherein said first radioisotope is ³²P; thereby forming a radioactive metal-labeled microsphere.

Also disclosed as reference is a method of preparing a radioactive microsphere, comprising the steps of:
combining a transition-metal, lanthanide or group 13-14 metal oxide, polyoxometalate, hydroxide, alkoxide, or carboxylate, or combination thereof and a microsphere comprising a hydrophilic polymer comprising a plurality of pendant moieties, thereby forming a metal-labeled microsphere; wherein said hydrophilic polymer comprises one or more polymerized monomers selected from the group consisting of N-[tris(hydroxymethyl)methyl]-acrylamide and vinylphosphonate; and wherein said pendant moieties are phosphonic acids; wherein said transition-metal, lanthanide or group 13-14 metal oxide, polyoxometalate, hydroxide, alkoxide, or carboxylate, or combination thereof is an oxide, polyoxometalate, hydroxide, alkoxide, or carboxylate of zirconium or combination thereof; and
combining said metal-labeled microsphere with a first radioisotope, wherein said first radioisotope is ³²P; thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and
converting the transition-metal, lanthanide or group 13-14 metal or combination thereof in the microsphere-metal complex to its oxide, polyoxometalate or hydroxide, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; said transition-metal, lanthanide or group 13-14 metal or combination thereof comprises a metal or combination thereof of zirconium, scandium, yttrium, lanthanum, hafnium, titanium, aluminum, silicon, gallium, indium, thallium, germanium, tin, lead, bismuth, tungsten, tantalum, cerium, praseodymium, neodymium, promethium, samarium, europium, gadolinium, terbium, dysprosium, holmium, erbium, thulium, ytterbium, or lutetium; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, wherein said first radioisotope is ⁹⁰Y, ³²P, ¹⁸F, ¹⁴⁰La, ¹⁵³Sm, ¹⁶⁵Dy, ¹⁶⁶Ho, ¹⁶⁹Er, ¹⁶⁹Yb, ¹⁷⁷Lu, ¹⁸⁶Re, ¹⁸⁸Re, ¹⁰³Pd, ¹⁹⁸Au, ¹⁹²Ir, ⁹⁰Sr, ¹¹¹In or ⁶⁷Ga; thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; wherein said hydrophilic polymer comprises one or more polymerized monomers selected from the group consisting of N-[tris(hydroxymethyl)methyl]-acrylamide and vinylphosphonate; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; wherein said transition-metal, lanthanide or group 13-14 metal is zirconium; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, wherein said first radioisotope is ³²P; thereby forming a radioactive metal-labeled microsphere.

Also disclosed as reference is a method of preparing a radioactive microsphere, comprising the steps of:
forming a microsphere from a polymer comprising a plurality of pendant moieties in the presence of a transition-metal, lanthanide or group 13-14 metal or combination thereof, thereby forming a microsphere-metal complex; wherein said hydrophilic polymer comprises one or more polymerized monomers selected from the group consisting of N-[tris(hydroxymethyl)-methyl]acrylamide and vinylphosphonate; and wherein said pendant moieties are phosphonic acids; and wherein said transition-metal, lanthanide or group 13-14 metal is zirconium; and
converting the transition-metal, lanthanide or group 13-14 metal or combination thereof in the microsphere-metal complex to its oxide, polyoxometalate, hydroxide, alkoxide, or carboxylate, thereby forming a metal-labeled microsphere; and
combining said metal-labeled microsphere with a first radioisotope, wherein said first radioisotope is ³²P; thereby forming a radioactive metal-labeled microsphere.

In certain embodiments, said microsphere is combined with the first radioisotope being in the form of ³²P phosphate at the site of treatment.

In certain embodiments, said microsphere is combined with a second radioisotope; wherein the atomic number of the first radioisotope is not the same as the atomic number of the second radioisotope.

In certain embodiments, said microsphere is combined with a second radioisotope at the site of treatment.

In certain embodiments, said microsphere is combined with the first radioisotope at the site of treatment.

In certain embodiments, said microsphere is combined with a second radioisotope; wherein the atomic number of the first radioisotope is not the same as the atomic number of the second radioisotope.

In certain embodiments, said microsphere is combined with a second radioisotope at the site of treatment.

Said microspheres may be administered using a catheter or a syringe.

Said microspheres may be administered by a catheter.

Also provided is a composition for use in a method of treating a mammal suffering from a head disorder, a neck disorder, a thorax disorders, an abdomenal disorder, a pelvic disorder, a cancer, cronic haemophilic synovitis, or arthritis; comprising the step of administering a composition comprising radioactive metal-labeled microsphere; wherein said hydrophilic polymer comprises one or more polymerized monomers selected from the group consisting of N-[tris(hydroxymethyl)methyl]acrylamide and vinylphosphonate; and wherein said pendant moieties are phosphonic acids; and wherein said transition-metal, lanthanide or group 13-14 metal is zirconium; and wherein said first radioisotope is in the form of ³²P phosphate.

Said microspheres may be used in the treatment of cancer, synovectomy, or arthritis.

Said microspheres may be used in the treatment of cancer.

Also disclosed as reference is a method of preparing a radioactive microsphere, comprising the steps of:
forming a microsphere comprising a hydrophilic polymer comprising a plurality of pendant moieties; and
combining said microsphere with a first radioisotope, thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and
combining said microsphere with a first radioisotope, thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and
combining said microsphere with a first radioisotope, thereby forming a radioactive microsphere.

Also disclosed as reference is method of preparing a radioactive microsphere, comprising the steps of:
forming a microsphere comprising a hydrophilic polymer comprising a plurality of pendant moieties; and
combining said microsphere with a first radioisotope, wherein said first radioisotope is ⁹⁰Y, ³²P, ¹⁸F, ¹⁴⁰La, ¹⁵³Sm, ¹⁶⁵Dy, ¹⁶⁶Ho, ¹⁶⁹Er, ¹⁶⁹Yb, ¹⁷⁷Lu, ¹⁸⁶Re, ¹⁸⁸Re, ¹⁰³Pd, ¹⁹⁸Au, ¹⁹²Ir, ⁹⁰Sr, ¹¹¹In or ⁶⁷Ga; thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said hydrophilic polymer comprises one or more polymerized monomers selected from the group consisting of crosslinked gelatin, oxidized starch, alginate, gellan, gum arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines;
combining said microsphere with a first radioisotope, wherein said first radioisotope is ⁹⁰Y, ³²P, ¹⁸F, ¹⁴⁰La, ¹⁵³Sm, ¹⁶⁵Dy, ¹⁶⁶Ho, ¹⁶⁹Er, ¹⁶⁹Yb, ¹⁷⁷Lu, ¹⁸⁶Re, ¹⁸⁸Re, ¹⁰³Pd, ¹⁹⁸Au, ¹⁹²Ir, ⁹⁰Sr, ¹¹¹In or ⁶⁷Ga; thereby forming a radioactive microsphere.

Also disclosed as reference is a method of preparing a radioactive microsphere, comprising the steps of:
forming a microsphere comprising a hydrophilic polymer comprising a plurality of pendant moieties, wherein said hydrophilic polymer comprises one or more polymerized monomers selected from the group consisting of crosslinked gelatin, N-[tris(hydroxymethyl)-methyl]acrylamide and vinylphosphonate; and
combining said microsphere with a first radioisotope, thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said pendant moieties are selected independently from the group consisting of phosphonic acids, bisphosphonic acids, sulfonic acids, and carboxylic acids; and
combining said microsphere with a first radioisotope, thereby forming a radioactive microsphere.

Also disclosed as reference is a method of preparing a radioactive microsphere, comprising the steps of:
forming a microsphere comprising a hydrophilic polymer comprising a plurality of pendant moieties; and
combining said microsphere with a first radioisotope, wherein said first radioisotope is, ⁹⁰Y, ⁴⁰La, ¹⁶⁹Yb, ¹¹¹In or ⁶⁷Ga; thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said hydrophilic polymer comprises one or more polymerized monomers selected from the group consisting of crosslinked gelatin, N-[tris(hydroxymethyl)-methyl]acrylamide and vinylphosphonate; and wherein said pendant moieties are selected independently from the group consisting of phosphonic acids, bisphosphonic acids, sulfonic acids, and carboxylic acids; and
combining said microsphere with a first radioisotope, wherein said first radioisotope is, ⁹⁰Y, ¹⁴⁰La, ¹⁶⁹Yb, ¹¹¹In or ⁶⁷Ga; thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said pendant moieties are phosphonic acids; and
combining said microsphere with a first radioisotope, thereby forming a radioactive microsphere.

Also disclosed as reference is a method of preparing a radioactive microsphere, comprising the steps of:
forming a microsphere comprising a hydrophilic polymer comprising a plurality of pendant moieties; and
combining said microsphere with a first radioisotope, wherein said first radioisotope is ⁹⁰Y, ¹¹¹In or ¹⁶⁶Ho; thereby forming a radioactive microsphere.

Also disclosed as reference is a method of preparing a radioactive microsphere, comprising the steps of:
forming a microsphere comprising a hydrophilic polymer comprising a plurality of pendant moieties; wherein said hydrophilic polymer comprises one or more polymerized monomers selected from the group consisting of N-[tris(hydroxymethyl)methyl]-acrylamide and vinylphosphonate; and wherein said pendant moieties are phosphonic acids; and
combining said microsphere with a first radioisotope, wherein said first radioisotope is ⁹⁰Y, ¹¹¹In or ¹⁶⁶Ho; thereby forming a radioactive microsphere.

Said microsphere may be combined with a first radioisotope at the site of treatment.

Said microsphere may be combined with a second radioisotope; wherein the atomic number of the first radioisotope is not the same as the
atomic number of the second radioisotope.

Said microsphere may be combined with a second radioisotope at the site of treatment.

Said microspheres may be administered using a catheter or a syringe.

Said microspheres may be administered by a catheter.

Said microspheres may be used in the treatment of cancer, synovectomy, or arthritis.

Said microspheres may be used in the treatment of cancer.

### Exemplification

The invention now being generally described, it will be more readily understood by reference to the following examples, which are included merely for purposes of illustration of certain aspects and embodiments of the present invention.

### Example 1

A mixture of 0.24 g of sorbitan sesquioleate in 350 mL of mineral oil was warmed to 60 °C in a stirred vessel. A gelatin solution was prepared by dissolving 20 g of porcine gelatin in 80 mL of a 60 °C aqueous 50 mM 2-morpholinoethanesulfonate (MES) buffer, previously adjusted to pH 5.5. The gelatin solution was added to the warmed, stirred oil, and the mixture was slowly cooled to 4 °C with stirring, and poured into cold water containing some detergent. The mixture was placed in a 4 °C refrigerator overnight. The oil was decanted away, and the gelatin microspheres in the remaining aqueous solution were placed in a stirred vessel at 4 °C, and treated with a solution of 0.6 g of EDC in about 15 mL of 50 mM MES buffer (pH 5.5). The mixture was stirred overnight at 4 °C and finally washed with several portions of room temperature water.

A portion of about 10 mL of microspheres in water (total volume about 20 mL) was added to 20 mL of zirconium acetate solution (Aldrich product 41,380-1 used as received, about 15 % Zr by weight). The beads were initially buoyant, but eventually settled to the bottom of the vessel. An additional 5 mL of zirconium acetate solution was added, and the mixture was allowed to stand for about 15 minutes. A final portion of 5 mL of zirconium acetate solution was added, and the mixture was allowed to stand for about 2 hours. The supernatant was decanted away from the microspheres, and the microspheres were washed four times with 50 mL portions of water. To the microspheres was added about 100 mL of 3% aqueous ammonia, and the mixture was allowed to stand overnight at room temperature. The microspheres were finally washed four times with water.

A 2-mL portion of settled microspheres in water (total volume of 6 mL) was treated with 2.5 g of a 3.09 % aqueous solution of Na₂HPO₄. The mixture was gently agitated for 1 hour at room temperature, and the supernatant was decanted away from the microspheres. The microspheres were washed five times with water; the total volume of the washes was about 30 mL. The washings were added to the supernatant, and phosphate was determined by precipitating MgNH₄PO₄ · 6H₂O (a standard procedure for phosphate analysis). No precipitate was isolable by filtration, although a small amount of finely divided, suspended precipitate was visible in the filtrate. This demonstrates that the microspheres absorbed almost all of the phosphate in the original solution, about 50 mg of phosphate ion (as PO₄).

### Example 2

Gelatin microspheres were prepared in a manner similar to that described in Example 1, and a 2-mL portion of the microspheres were treated twice with zirconium acetate solution. The microspheres were washed with water and treated for about 2 hours with 3 % aqueous ammonia. The microspheres were washed several times with water. A 1-mL portion of the microspheres was treated with 5.21 g of 5.66 % aqueous Na₂HPO₄ and gently agitated for one hour. The supernatant was decanted away, and the beads were washed 5 times with 10 mL portions of water. Phosphate analysis of the combined supernatant and washes showed that 20 % of the phosphate, or 39 mg of PO₄, was absorbed by the 1-mL portion of microspheres.

### Example 3

### Hydrogel Microsphere Preparation by Suspension Polymerization

Microspheres were prepared according to the general procedure described below, using the monomers sodium acrylate (NaA), ethylene glycol methacrylate phosphate (EGMP), vinylphosphonic acid (VPh), and N-[tris(hydroxymethyl)methyl]acrylamide (trisacryl, TA), according to the following table:

| **Sample** | **Monomer 1** | **Monomer 2** |
|---|---|---|
| NaA | NaA, 100.0 g | - |
| NaA/TA | NaA, 10.8 g | TA, 89.2 g |
| EGMP | EGMP, 100.0 g | - |
| EGMP/TA | EGMP, 10.8 g | TA, 89.2 g |
| VPh(10)/TA | VPh, 10.8 g | TA, 89.2 g |
| VPh(1)/TA | VPh, 1.1 g | TA, 98.9 g |
| TA | TA, 100.0 g | - |

A 4-liter Morton-type reaction vessel, equipped with an overhead stirrer, was charged with 3.2 L of mineral oil, 2.4 g of sorbitan sesquioleate, and 3.2 mL of N,N,N',N'-tetramethylethylenediamine, and the solution was warmed to 60 °C under a nitrogen atmosphere. In about 650 mL of water was dissolved 100 g of monomer (see table above) and 8.0 g of N,N'-methylenebisacrylamide. For those preparations where EGMP or VPh was included in the monomer solution, aqueous sodium hydroxide was added to adjust the pH to about 6. Water was added to adjust the volume to 800 mL, and the mixture was warmed to 60 °C. To the aqueous solution was added 1.1 g of ammonium persulfate in about 15 mL of water. The mixture was briefly stirred to achieve homogeneity, and added with vigorous stirring to the warmed oil solution. The mixture was maintained at 60 °C under a nitrogen atmosphere with vigorous stirring. Polymerization was evidenced by a mild exotherm of 3 - 5 °C. The mixture was stirred for about one hour, and the microspheres were isolated by repeated washing with water, to eliminate the oil. To the mixture of microspheres and excess water was added 0.9 % by weight of sodium chloride. The microspheres were stored in the 0.9 % sodium chloride solution.

Zirconium acetate solutions were prepared by adding 0.75 mL (solution 1), 7.5 mL (solution 2), or 75.0 mL (solution 3) of zirconium acetate solution (Aldrich product 41,380-1) to 750 mL of 10 % aqueous acetic acid. Each of a sample of 100 mL of microspheres was treated for three hours with 100 mL of each zirconium acetate solution, washed four times with water, treated for one hour with 100 mL of 3 % aqueous ammonia, and finally washed four times with water and four times with 0.9 % aqueous sodium chloride.

The final product is identified by the monomer composition and the zirconium acetate solution that were used in the preparation. The suffix -0 indicates microspheres that were not treated with zirconium acetate solution. For example, EGMP-0 indicates the microsphere composition prepared from 100.0 g of EGMP, but not treated with zirconium acetate, while EGMP/TA-3 indicates the microsphere composition prepared from 10.8 g of EGMP, 89.2 g of TA, and treated with zirconium acetate solution 3.

### Example 4

### Phosphate Absorption

A sample of about 3 mL of microspheres (from Example 3), freshly washed with 0.9 % sodium chloride solution, was added to a 15-mL centrifuge tube, and centrifugated for five minutes at a force of about 140 times gravity. Microspheres and supernatant solution were removed so that the tube contained 2.0 mL of compacted microspheres in a total volume (microspheres + supernatant) of 5.0 mL. The microspheres were resuspended by shaking the tube, and 25 microliters of 0.18 % aqueous Na₂HPO₄ was added, to give a calculated concentration of 6.0 parts per million (ppm), or 30 micrograms, of PO₄²⁻ in the mixture. If all of this phosphate were composed of ³²P, the radioactivity would be about 2.9 curies. The tube was gently tumbled for 10 minutes, and centrifugated for 5 minutes. An aliquot of the supernatant was removed for phosphate analysis. The phosphate was determined by a standard photometric method based on formation of phosphomolybdic acid. The results show that treating the microspheres with higher concentrations of zirconium results in higher phosphate absorption from solution. The microspheres prepared with the highest concentration of zirconium (solution 3 from example 3) absorb essentially all of the phosphate. Entries 15 and 16 demonstrate that the zirconium treatment is necessary for the microspheres to absorb phosphate from solution.

| **Entry** | **Microsphere sample (from Example 3)** | **Residual phosphate (ppm)** |
|---|---|---|
| 1 | NaA-1 | 4.1 |
| 2 | NaA-2 | 0.1 |
| 3 | NaA-3 | < 0.1 |
| 4 | NaA/TA-1 | 1.0 |
| 5 | NaA/TA-2 | < 0.1 |
| 6 | NaA/TA-3 | < 0.1 |
| 7 | EGMP-1 | 2.0 |
| 8 | EGMP-2 | 0.1 |
| 9 | EGMP-3 | < 0.1 |
| 10 | EGMP/TA-1 | 2.9 |
| 11 | EGMP/TA-2 | 0.2 |
| 12 | EGMP/TA-3 | < 0.1 |
| 13 | VPh(1)/TA-3 | 0.1 |
| 14 | VPh(10)/TA-3 | 0.2 |
| 15 | VPh(10)/TA-0 | 6.2 |
| 16 | TA-0 | 5.7 |

### Reference example 5

### Yttrium, Lanthanum, and Ytterbium Ion Absorption

Samples of EGMP and EGMP/TA microspheres from Example 3 were treated as for Example 4, except, instead of phosphate solution, the microspheres were treated with 25 microliters of certified standard 1000 ppm metal ion solutions. The concentration of metal ion is calculated to be 5.0 ppm in the mixture. The metal ion was either yttrium, lanthanum, or ytterbium. The remaining metal ion concentration in the supernatant was analyzed by a standard photometric method based on complexation with the dye Arsenazo-III. The results are summarized in the following table. Comparing entries 9 and 10, the results show that the microspheres prepared with the lower amount of vinyl phosphonate require the zirconium treatment for efficient metal ion absorption. The other microspheres efficiently absorb the metal ions, independently of the amount of zirconium acetate that was used in their preparation.

| | | **Residual Metal Ion (ppm)** | | |
|---|---|---|---|---|
| **Entry** | **Microsphere sample (from Example 3)** | **Yttrium** | **Lanthanum** | **Ytterbium** |
| 1 | EGMP-0 | 0.1 | 0.2 | 0.3 |
| 2 | EGMP-1 | 0.1 | 0.3 | 0.3 |
| 3 | EGMP-2 | 0.2 | 0.3 | 0.3 |
| 4 | EGMP-3 | 0.1 | 0.1 | 0.2 |
| 5 | EGMP/TA-0 | 0.2 | 0.2 | 0.3 |
| 6 | EGMP/TA-1 | 0.3 | 0.4 | 0.3 |
| 7 | EGMP/TA-2 | 0.1 | 0.2 | 0.2 |
| 8 | EGMP/TA-3 | 0.2 | 0.2 | 0.2 |
| 9 | VPh(1)/TA-0 | 2.0 | 0.8 | 0.3 |
| 10 | VPh(1)/TA-3 | 0.2 | 0.4 | 0.3 |
| 11 | VPh(10)/TA-0 | 0.2 | 0.2 | 0.3 |
| 12 | VPh(10)/TA-3 | 0.1 | 0.1 | 0.3 |

### Reference example 6

### Gallium and Indium Ion Absorption

A sample of 2 mL of EGMP-3 microspheres in 0.9 % aqueous sodium chloride (5 mL mixture volume) was prepared as for Example 5, except it was treated with 250 microliters of certified standard 1000 ppm gallium solution. The metal ion concentration is higher in this example, compared to those in Example 5, because the Arsenazo-III method is less sensitive for gallium. The concentration of gallium is calculated to be 48 ppm in the mixture. The gallium concentration in the supernatant was lower than the limit of detection by the Arsenazo-III complexation method, showing that most of the gallium was absorbed by the microspheres.

The Arsenazo-III method is not useful for indium analysis. A qualitative method, based on precipitation by 8-hydroxyquinoline, was used. A portion of 50 mg of 8-hydroxyquinoline was dissolved in 20 mL of 10 % aqueous acetic acid, and 4 N aqueous sodium metal hydroxide was slowly added until the pH was 6.5. A sample of EGMP-3 microspheres (2 mL of microspheres in 0.9 % aqueous sodium chloride, for a total volume of 5 mL) was treated with 250 microliters of 1000 ppm standard indium solution for 15 minutes. For comparison, a blank solution of 250 microliters of the standard indium solution in 5 mL of 0.9 % aqueous sodium chloride was prepared. A 2-mL portion of the supernatant from the microsphere suspension and a 2-mL portion of the comparison solution were each added to separate 5-mL portions of the 8-hydroxyquinoline solution. The 8-hydroxyquinoline solution treated with the comparison solution immediately developed visible turbidity, while that treated with the microsphere supernatant did not. This result qualitatively shows that the microspheres absorb at least part of the indium from a 48 ppm solution.

### Example 7

### Radioisotope absorption

A 2-mL volume of settled microspheres in 0.9 % aqueous sodium chloride solution (5.0 mL total volume) is treated with a 1-mL aqueous radioisotope-containing solution of known radioactivity (see the table below). The mixture is gently agitated for about 15 minutes, and the microspheres are washed several times with 0.9 % aqueous sodium chloride. The final radioactivity of the microspheres is then determined.

The results show that all of the microsphere compositions efficiently absorb the radioactive metal ions from solution. For the isotopes Y-90 and Ho-166 (beta emitters), the radioactivity of the microspheres is comparable to that currently used for internal radiotherapy. For In-111 (gamma emitter), the absorbed radioactivity is sufficient for diagnostic imaging by gamma camera. These radioactive microspheres are therefore useful for nuclear medicine and diagnostic imaging.

The results also show that all of the zirconium-containing microsphere compositions efficiently absorb radioactive phosphate in therapeutically useful amounts, but those compositions lacking zirconium do not. Therefore, if it is desirable to treat the patient with radiophosphate, the zirconium-containing compositions are required. The zirconium is not required if the microspheres are not to be labeled with radiophosphate .

| **Microsphere Composition** | **Radioisotope** | **Initial Solution Radioactivity (mCi)** | **Final Microsphere Radioactivity (mCi)** |
|---|---|---|---|
| EGMP-0 | P-32 phosphate | 100 | <10 |
| EGMP/TA-0 | P-32 phosphate | 100 | <10 |
| VPh(10)/TA-0 | P-32 phosphate | 100 | <10 |
| EGMP-3 | P-32 phosphate | 100 | >90 |
| EGMP/TA-3 | P-32 phosphate | 100 | >90 |
| VPh(10)/TA-3 | P-32 phosphate | 100 | >90 |
| EGMP-0 | Y-90 (+3 ion) | 100 | >90 |
| EGMP/TA-0 | Y-90 (+3 ion) | 100 | >90 |
| VPh(10)/TA-0 | Y-90 (+3 ion) | 100 | >90 |
| EGMP-3 | Y-90 (+3 ion) | 100 | >90 |
| EGMP/TA-3 | Y-90 (+3 ion) | 100 | >90 |
| VPh(10)/TA-3 | Y-90 (+3 ion) | 100 | >90 |
| EGMP-0 | In-111 (+3 ion) | 1.0 | >0.9 |
| EGMP/TA-0 | In-111 (+3 ion) | 1.0 | >0.9 |
| VPh(10)/TA-0 | In-111 (+3 ion) | 1.0 | >0.9 |
| EGMP-3 | In-111 (+3 ion) | 1.0 | >0.9 |
| EGMP/TA-3 | In-111 (+3 ion) | 1.0 | >0.9 |
| VPh(10)/TA-3 | In-111 (+3 ion) | 1.0 | >0.9 |
| EGMP-0 | Ho-166 (+3 ion) | 100 | >90 |
| EGMP/TA-0 | Ho-166 (+3 ion) | 100 | >90 |
| VPh(10)/TA-0 | Ho-166 (+3 ion) | 100 | >90 |
| EGMP-3 | Ho-166 (+3 ion) | 100 | >90 |
| EGMP/TA-3 | Ho-166 (+3 ion) | 100 | >90 |
| VPh(10)/TA-3 | Ho-166 (+3 ion) | 100 | >90 |

### Labelled Microspheres 8

### Absorption of Radioisotope Mixtures

Internal radiation therapy using microspheres comprising beta-emitting isotopes, such as Y-90 or P-32, is useful because of the limited depth of penetration of the radiation, which spares most of the healthy tissue from its harmful effects. On the other hand, this also requires that the microspheres be deposited inside of, or in the immediate vicinity of, the diseased tissue. It would therefore be desirable to perform accurate dosimetry after the microspheres have been delivered into the patient, to determine if, in fact, the microspheres were deposited in a manner sufficient to kill the diseased tissue. Portions of diseased tissue that eluded a lethal radiation dose ("cold spots") could thereby be detected, and retreatment would be indicated. The currently-available microspheres for internal radiation therapy comprise Y-90, which emits only beta radiation. These microspheres therefore cannot be imaged by gamma camera, precluding accurate dosimetry after the patient has been treated. This example shows that the microspheres can be simultaneously labeled with beta- and gamma-emitting radioisotopes. The beta-emitter (such as radiophosphate or Y-90) is absorbed in a therapeutically useful amount, and the gamma-emitter (such as indium-111) is absorbed in an amount imageable by gamma camera.

### Labelled microspheres 9

### P-32 and In-Ill

A 2-mL volume of settled VPh(10)/TA-3 microspheres in 0.9% aqueous sodium chloride solution (5.0 mL total volume) are treated for 15 minutes with a 1-mL aqueous solution containing about 100 millicuries of radiophosphate. The microspheres are washed several times with 0.9% aqueous sodium chloride. The microspheres are then treated for 15 minutes with a 1-mL aqueous solution containing about 1 millicurie of In-111 (as its 3+ ion). The microspheres are finally washed several times with 0.9% aqueous sodium chloride, and the absorbed radioactivity of the microspheres is determined. Radioactivity measurements on the microspheres show that they absorb greater than 90% of both isotopes. The microspheres are therefore useful for internal radiotherapy, and can also be imaged by gamma camera.

### Reference Labelled microspheres 10

### Y-90 and In-111

A 2-mL volume of settled VPh(10)/TA-0 microspheres in 0.9% aqueous sodium chloride solution (5.0 mL total volume) are treated for 15 minutes with a 1-mL aqueous solution containing about 100 millicuries of Y-90 (as its 3+ ion). The microspheres are washed several times with 0.9% aqueous sodium chloride. The microspheres are then treated for 15 minutes with a 1-mL aqueous solution containing about 1 millicurie of In-111 (as its 3+ ion). Alternatively, the microspheres can be treated with an aqueous solution containing both of these radioisotopes. The microspheres are finally washed several times with 0.9 % aqueous sodium chloride, and the absorbed radioactivity of the microspheres is determined. Radioactivity measurements on the microspheres show that they absorb greater than 90% of both isotopes. The microspheres are therefore useful for internal radiotherapy, and can also be imaged by gamma camera. Similar results are obtained with VPh(10)/TA-3 microspheres.

### Example 11

### Preparation of Zirconia-Impregnated Hydrogel Microspheres

Zirconia-impregnated hydrogel microspheres were produced using suspension polymerization carried out in a 4-liter glass Morton-type vessel, equipped with a mechanical overhead stirrer and external jacket for temperature control by recirculating fluid. The vessel was charged with 3.2 liters of mineral oil and 2.4 mL of sorbitan sesquioleate, and the contents were warmed to 44 °C. An aqueous monomer mixture was prepared separately by adding 100 g of trisacryl, 8.0 g ofN,N'-methylenebisacrylamide, 5.0 mL of a commercial colloidal zirconia preparation (Nyacol Zr 100/20, used as received), and 10.0 mL of glacial acetic acid to about 600 mL of water, and water was used to adjust the volume of the mixture to 800 mL. To the aqueous mixture was added a solution of 2.0 g of a water-soluble azo initiator (VA-044, Wako Chemicals USA, Richmond, VA, USA) in a few milliliters of water. The mixture was thoroughly mixed at room temperature and added in a single portion to the warmed, vigorously agitated mineral oil. No polymerization was evident after 2 hours. The external heating fluid was warmed to 75 °C, and when the temperature of the contents reached 65 °C, polymerization was evidenced by a mild exotherm. The external temperature was maintained at 75 °C for an additional 2 hours after the exotherm, and the contents of the vessel were drained into 4 liters of water. The mineral oil was decanted away after the layers separated. The microspheres were washed several times with water and with 0.9 % aqueous sodium chloride (saline). Microspheres in the range of about 100 to 300 µm were isolated by sieving.

### Example 12

### Incorporation of ³²P into Zirconia-Impregnated Hydrogel Microspheres

Phosphorous-binding properties of zirconium were used to make radioactive zirconia microspheres. Schafer WA et al. Physical and chemical characterization of a porous phosphate-modified zirconia substrate. Journal of Chromatography A Dec 1991;587(2):137-147. The microspheres, suspended in water and alcohol, were washed once with NaOH (1M) for 1 minute, and seven times with purified water, using centrifugation for 60 seconds at 6000 rpm between washes, until the pH of the solution reached 7.0. Microspheres were resuspended in 1 mL of saline. An aliquot of the spheres was removed to determine density, using a hemacytometer. The sphere density of the pool was 1.75x10⁶ spheres/mL of solution. Two other samples of 100 and 10 µl of the pool solution were centrifuged, dehydrated with a Speed Vac, and spheres were weighted.

The ³²P uptake and leaching of zirconia beads was studied in vitro. Two different concentrations of microspheres and 2 different concentrations of ³²P were used for the experiments. Two aliquot parts of 100 µl and 2 aliquot parts of 10 µl were removed from this pool solution of microspheres, diluted with saline, and a given quantity of ³²P solution (Perkin-Elmer Life Sciences, Boston, MA, USA) was added to each of these microspheres solutions. See Table 1.

**Table 1**

| Uptake of ³²P from zirconia beads | | | | | | |
|---|---|---|---|---|---|---|
| Samples | ³²P volume | Spheres solution volume | Initial ³²P activity | Final ³²P activity on spheres | Spheres number | Activity per sphere |
| | (µl) | (µl) | (µCi) | (µCi) | | (nCi) |
| A | 100 | 100 | 87±8 | 10.54 | 175000 | 0.060 |
| B | 100 | 10 | 87±8 | 1.49 | 17500 | 0.085 |
| C | 10 | 100 | 11±1 | 1.95 | 175000 | 0.011 |
| D | 10 | 10 | 11±1 | 0.15 | 17500 | 0.009 |

Microspheres were incubated 40 minutes in the ³²P solution, and washed twice with saline. For each sample, 3 elutions with fresh saline (20 minutes each) were done on a vibrating table. Samples were centrifuged for 60 seconds at 6000 rpm, and the supernatant was removed. Each sample was then washed with 1 mL of saline and agitated for 10 seconds. Final solutions were disposed in vials with 20 mL of scintillation liquid each, and counted (Tri-Carb, Packard). Higher activity per sphere, 0.085 nCi/sphere was obtained with 100 µl of ³²P solution and 10 µl of spheres solution (approximately 17500 spheres); in this sample, the final ³²P activity was 1.49 µCi for 1.1 mg of spheres (1.35 µCi/mg).

## Claims

1. A microsphere, comprising a hydrophilic polymer comprising a plurality of pendant moieties; a zirconium oxide, polyoxometalate, hydroxide, alkoxide, carboxylate or combination thereof; and a first radioisotope, wherein said first radioisotope is in the form of ³²P phosphate.

2. The microsphere of claim 1, further comprising a second radioisotope; wherein the atomic number of the first radioisotope is not the same as the atomic number of the second radioisotope.

3. The microsphere of claim 1 or 2, wherein said hydrophilic polymer comprises one or more polymerized monomers selected from
(a) the group consisting of acrylics, vinyls, acetals, allyls, cellulosics, methacrylates, polyamides, polycarbonate, polyesters, polyimide, polyolefins, polyphosphates, polyurethanes, silicones, styrenics, and polysaccharides;
(b) the group consisting of crosslinked gelatin, oxidized starch, alginate, gellan, gum Arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide;
(c) the group consisting of crosslinked gelatin, N-[tris(hydroxymethyl)methyl]acrylamide and vinylphosphonate; or
(d) the group consisting of N-[tris(hydroxymethyl)-methyl]acrylamide and vinylphosphonate.

4. The microsphere of claim 1 or 2, wherein said pendant moieties are selected independently from
(a) the group consisting of phosphonic acids, phosphates, bisphosphonic acids, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines;
(b) the group consisting of phosphonic acids, bisphosphonic acids, sulfonic acids, and carboxylic acids; or
(c) phosphonic acids.

5. The microsphere of claim 2, wherein said second radioisotope is (a) Tc-99m, ¹¹¹In or ⁶⁷Ga; or (b) ¹¹¹In.

6. The microsphere of claim 2, wherein said second radioisotope is ¹¹¹In.

7. The microsphere of claim 1 or 2, wherein said hydrophilic polymer comprises one or more polymerized monomers selected from
(a) the group consisting of crosslinked gelatin, oxidized starch, alginate, gellan, gum Arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; or
(b) the group consisting of crosslinked gelatin, N-[tris(hydroxymethyl)methyl]acrylamide and vinylphosphonate; and wherein said pendant moieties are selected independently from the group consisting of phosphonic acids, bisphosphonic acids, sulfonic acids, and carboxylic acids; or
(c) the group consisting of N-[tris(hydroxymethyl)methyl]-acrylamide and vinylphosphonate; and wherein said pendant moieties are phosphonic acids; or
(d) the group consisting of crosslinked gelatin, oxidized starch, alginate, gellan, gum Arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; and wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; and wherein said second radioisotope is Tc-99m, ¹¹¹In or ⁶⁷Ga; or
(e) the group consisting of crosslinked gelatin, N-[tris(hydroxymethyl)-methyl]acrylamide and vinylphosphonate; and wherein said pendant moieties are selected independently from the group consisting of phosphonic acids, bisphosphonic acids, sulfonic acids, and carboxylic acids; and wherein the second radioisotope is Tc-99m, ¹¹¹In or ⁶⁷Ga; or
(f) the group consisting of N-[tris(hydroxymethyl)methyl]-acrylamide and vinylphosphonate; and wherein said pendant moieties are phosphonic acids; and wherein said second radioisotope is Tc-99m, ¹¹¹In or ⁶⁷Ga; or
(g) the group consisting of N-[tris(hydroxymethyl)methyl]-acrylamide and vinylphosphonate; and wherein said pendant moieties are phosphonic acids; and wherein said second radioisotope is ¹¹¹1n.

8. The microsphere of claim 2, wherein the ratio of the radioactivity of the second radioisotope to the first radioisotope is in the range from about 1:10 to about 1:10⁷ at the time of use; or in the range from about 1:10² to 1:10⁶ at the time of use; or in the range from about 1:10⁴ to 1:10⁵ at the time of use; or in the range from about 1:10 to 1:10³ at the time of use.

9. The microsphere of claim 2, wherein said first radioisotope is not leached from said microsphere to an extent greater than about 3%; wherein said second radioisotope is not leached from said microsphere to an extent greater than about 3%; or to an extent greater than about 1%; wherein said second radioisotope is not leached from said microsphere to an extent greater than about 1%.

10. The microsphere of claim 1 or 2, wherein said microsphere further comprises (a) a biologically active agent; or (b) a contrast-enhancing agent; optionally wherein said contrast-enhancing agent is selected from the group consisting of radiopaque materials, paramagnetic materials, heavy atoms, transition metals, lanthanides, actinides, and dyes.

11. The microsphere of claim 1 or 2, wherein the diameter of said microsphere is in the range from about 1-2000 micrometers, about 1-1000 micrometers, about 1-500 micrometers, about 1-100 micrometers, or about 10-40 micrometers.

12. A method of preparing a radioactive microsphere, comprising the steps of:
combining zirconium and a microsphere comprising a hydrophilic polymer comprising a plurality of pendant moieties, thereby forming a microsphere-metal complex; and
converting the zirconium in the microsphere-metal complex to its oxide, polyoxometalate, hydroxide, alkoxide or carboxylate, thereby forming a metal-labeled microsphere; and
combining said metal-labeled microsphere with a first radioisotope, wherein said radioisotope is in the form of ³²P phosphate, thereby forming a radioactive metal-labeled microsphere.

13. A method according to claim 12, wherein said hydrophilic polymer comprises one or more polymerized monomers selected from
(a) the group consisting of crosslinked gelatin, oxidized starch, alginate, gellan, gum Arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; or
(b) the group consisting of crosslinked gelatin, N-[tris(hydroxymethyl)-methyl]acrylamide and vinylphosphonate.

14. A method according to claim 12 or 13, wherein said pendant moieties are selected independently from
(a) the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; or
(b) the group consisting of phosphonic acids, bisphosphonic acids, sulfonic acids, and carboxylic acids.

15. A method according to claim 12, comprising the steps of:
combining zirconium and a microsphere comprising a hydrophilic polymer comprising a plurality of pendant moieties, thereby forming a microsphere-metal complex; wherein said hydrophilic polymer comprises one or more polymerized monomers selected from the group consisting of crosslinked gelatin, N-[tris(hydroxymethyl)-methyl]acrylamide and vinylphosphonate; and wherein said pendant moieties are selected independently from the group consisting of phosphonic acids, bisphosphonic acids, sulfonic acids, and carboxylic acids; and
converting the zirconium in the microsphere-metal complex to its oxide, polyoxometalate, hydroxide, alkoxide, or carboxylate, thereby forming a metal-labeled microsphere; and
combining said metal-labeled microsphere with a first radioisotope, wherein said first radioisotope is in the form of ³²P phosphate; thereby forming a radioactive metal-labeled microsphere.

16. A method according to claim 12 wherein said hydrophilic polymer comprises one or more polymerized monomers selected from the group consisting of N-[tris(hydroxymethyl)methyl]-acrylamide and vinylphosphonate; and wherein said pendant moieties are phosphonic acids.

17. A method of preparing a radioactive microsphere, comprising the steps of:
combining a zirconium oxide, polyoxometalate, hydroxide, alkoxide, or carboxylate, or a combination thereof and a microsphere comprising a hydrophilic polymer comprising a plurality of pendant moieties, thereby forming a metal-labeled microsphere; and
combining said metal-labeled microsphere with a first radioisotope, wherein said first radioisotope is in the form of ³²P phosphate, thereby forming a radioactive metal-labeled microsphere.

18. A method according to claim 17, wherein said hydrophilic polymer comprises one or more polymerized monomers selected from
(a) the group consisting of crosslinked gelatin, oxidized starch, alginate, gellan, gum Arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; or
(b) the group consisting of N-[tris(hydroxymethyl)methyl]-acrylamide and vinylphosphonate.

19. A method according to claim 17 or 18, wherein said pendant moieties are selected independently from
(a) the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines; or
(b) phosphonic acids.

20. A method of preparing a radioactive microsphere, comprising the steps of:
forming a microsphere from a polymer comprising a plurality of pendant moieties in the presence of zirconium thereby forming a microsphere-metal complex; and
converting the zirconium in the microsphere-metal complex to its oxide, polyoxometalate, hydroxide, alkoxide, or carboxylate, thereby forming a metal-labeled microsphere; and
combining said metal-labeled microsphere with a first radioisotope, wherein said first radioisotope is in the form of ³²P phosphate, thereby forming a radioactive metal-labeled microsphere.

21. A method according to claim 20, wherein said hydrophilic polymer comprises one or more polymerized monomers selected from
(a) the group consisting of crosslinked gelatin, oxidized starch, alginate, gellan, gum Arabic, galactan, arabinogalactan, chitosan, hyaluronan, chondroitin sulfate, keratan sulfate, heparan sulfate, dermatan sulfate, carboxymethylcellulose, oxidized cellulose, acrylate, methacrylate, ethylene glycol methacrylate phosphate, vinylphosphonate, N-[tris(hydroxymethyl)methyl]-acrylamide, N,N'-methylene-bis-acrylamide, N',N'-diallyl-tartradiamide, and glyoxal-bis-acrylamide; or
(b) the group consisting of N-[tris(hydroxymethyl)methyl]-acrylamide and vinylphosphonate.

22. A method according to claim 20 or 21, wherein said pendant moieties are selected independently from the group consisting of phosphonic acids, bisphosphonic acids, phosphates, polyphosphates, diphosphates, triphosphates, sulfonic acids, sulfates, carboxylic acids, carbamic acids, hydroxamic acids, acyl hydrazides, thiols, amines, silicates, aluminates, titanates, zirconates, pyridines, imidazoles, thiphenes, thiazoles, furans, purines, pyrimidines, and hydroxyquinolines.

23. The method of any of claims 12 to 22, wherein said microsphere is combined with a second radioisotope and wherein the atomic number of the first radioisotope is not the same as the atomic number of the second radioisotope.

24. The method of any of claims 12 to 23, wherein said microspheres are suitable for administration using a catheter or a syringe.

25. A radioactive metal-labeled microsphere according to any of claims 1-11; wherein said hydrophilic polymer comprises one or more polymerized monomers selected from the group of N-[tris(hydroxymethyl)methyl]-acrylamide and vinylphosphonate; and wherein said pendant moieties are phosphonic acids; for treating a mammal suffering from a head disorder, a neck disorder, a thorax disorder, an abdominal disorder, a pelvic disorder, a cancer, chronic haemophilic synovitis, or arthritis.

26. A microsphere according to any one of claims 1 to 11 for the treatment of cancer, synovectomy, or arthritis.

## Patentansprüche

1. Mikrosphäre, die Folgendes umfasst: ein hydrophiles Polymer, das eine Vielzahl von anhängenden Einheiten umfasst; ein Zirconiumoxid, -polyoxometallat, -hydroxid, -alkoxid, -carboxylat oder eine Kombination davon; und ein erstes Radioisotop, wobei das erste Radioisotop in der Form von ³²P-Phosphat vorliegt.

2. Mikrosphäre nach Anspruch 1, die weiter ein zweites Radioisotop umfasst, wobei die Atomzahl des ersten Radioisotops nicht die gleiche ist wie die Atomzahl des zweiten Radioisotops.

3. Mikrosphäre nach Anspruch 1 oder 2, wobei das hydrophile Polymer ein oder mehrere polymerisierte Monomere umfasst, die aus Folgenden ausgewählt sind:
(a) der Gruppe, die aus Folgenden besteht: Acrylen, Vinylen, Acetalen, Allylen, Cellulosen, Methacrylaten, Polyamiden, Polycarbonat, Polyestern, Polyimid, Polyolefinen, Polyphosphaten, Polyurethanen, Siliconen, Styrolen und Polysacchariden;
(b) der Gruppe, die aus Folgenden besteht: vernetzter Gelatine, oxidierter Stärke, Alginat, Gellan, Gummiarabikum, Galactan, Arabinogalactan, Chitosan, Hyaluronsäure, Chondroitinsulfat, Keratansulfat, Heparansulfat, Dermatansulfat, Carboxymethylcellulose, oxidierter Cellulose, Acrylat, Methacrylat, Ethylenglycolmethacrylatphosphat, Vinylphosphonat, N-[Tris(hydroxymethyl)methyl]acrylamid, N,N'-Methylenbisacrylamid, N',N'-Diallylweinsäurediamid und Glyoxalbisacrylamid;
(c) der Gruppe, die aus vernetzter Gelatine, N-[Tris(hydroxymethyl)methyl]acrylamid und Vinylphosphonat besteht; oder
(d) der Gruppe, die aus N-[Tris(hydroxymethyl)methyl]acrylamid und Vinylphosphonat besteht.

4. Mikrosphäre nach Anspruch 1 oder 2, wobei die anhängenden Einheiten unabhängig aus Folgenden ausgewählt sind:
(a) der Gruppe, die aus Folgenden besteht: Phosphonsäuren, Phosphaten, Bisphosphonsäuren, Polyphosphaten, Diphosphaten, Triphosphaten, Sulfonsäuren, Sulfaten, Carbonsäuren, Carbaminsäuren, Hydroxamsäuren, Acylhydraziden, Thiolen, Aminen, Silicaten, Aluminaten, Titanaten, Zirconaten, Pyridinen, Imidazolen, Thiophenen, Thiazolen, Furanen, Purinen, Pyrimidinen und Hydroxychinolinen;
(b) der Gruppe, die aus Phosphonsäuren, Bisphosphonsäuren, Sulfonsäuren und Carbonsäuren besteht; oder
(c) Phosphonsäuren.

5. Mikrosphäre nach Anspruch 2, wobei das zweite Radioisotop (a) Tc-99m, ¹¹¹In oder ⁶⁷Ga oder (b) ¹¹¹In ist.

6. Mikrosphäre nach Anspruch 2, wobei das zweite Radioisotop ¹¹¹In ist.

7. Mikrosphäre nach Anspruch 1 oder 2, wobei das hydrophile Polymer ein oder mehrere polymerisierte Monomere umfasst, die aus Folgenden ausgewählt sind:
(a) der Gruppe, die aus Folgenden besteht: vernetzter Gelatine, oxidierter Stärke, Alginat, Gellan, Gummiarabikum, Galactan, Arabinogalactan, Chitosan, Hyaluronsäure, Chondroitinsulfat, Keratansulfat, Heparansulfat, Dermatansulfat, Carboxymethylcellulose, oxidierter Cellulose, Acrylat, Methacrylat, Ethylenglycolmethacrylatphosphat, Vinylphosphonat, N-[Tris(hydroxymethyl)methyl]acrylamid, N,N'-Methylenbisacrylamid, N',N'-Diallylweinsäurediamid und Glyoxalbisacrylamid; und wobei die anhängenden Einheiten unabhängig aus der Gruppe ausgewählt sind, die aus Folgenden besteht: Phosphonsäuren, Bisphosphonsäuren, Phosphaten, Polyphosphaten, Diphosphaten, Triphosphaten, Sulfonsäuren, Sulfaten, Carbonsäuren, Carbaminsäuren, Hydroxamsäuren, Acylhydraziden, Thiolen, Aminen, Silicaten, Aluminaten, Titanaten, Zirconaten, Pyridinen, Imidazolen, Thiophenen, Thiazolen, Furanen, Purinen, Pyrimidinen und Hydroxychinolinen; oder
(b) der Gruppe, die aus vernetzter Gelatine, N-[Tris(hydroxymethyl)methyl]acrylamid und Vinylphosphonat besteht; und wobei die anhängenden Einheiten unabhängig aus der Gruppe ausgewählt sind, die aus Phosphonsäuren, Bisphosphonsäuren, Sulfonsäuren und Carbonsäuren besteht; oder
(c) der Gruppe, die aus N-[Tris(hydroxymethyl)methyl]acrylamid und Vinylphosphonat besteht; und wobei die anhängenden Einheiten Phosphonsäuren sind; oder
(d) der Gruppe, die aus Folgenden besteht: vernetzter Gelatine, oxidierter Stärke, Alginat, Gellan, Gummiarabikum, Galactan, Arabinogalactan, Chitosan, Hyaluronsäure, Chondroitinsulfat, Keratansulfat, Heparansulfat, Dermatansulfat, Carboxymethylcellulose, oxidierter Cellulose, Acrylat, Methacrylat, Ethylenglycolmethacrylatphosphat, Vinylphosphonat, N-[Tris(hydroxymethyl)methyl]acrylamid, N,N'-Methylenbisacrylamid, N',N'-Diallylweinsäurediamid und Glyoxalbisacrylamid; und wobei die anhängenden Einheiten unabhängig aus der Gruppe ausgewählt sind, die aus Folgenden besteht: Phosphonsäuren, Bisphosphonsäuren, Phosphaten, Polyphosphaten, Diphosphaten, Triphosphaten, Sulfonsäuren, Sulfaten, Carbonsäuren, Carbaminsäuren, Hydroxamsäuren, Acylhydraziden, Thiolen, Aminen, Silicaten, Aluminaten, Titanaten, Zirconaten, Pyridinen, Imidazolen, Thiophenen, Thiazolen, Furanen, Purinen, Pyrimidinen und Hydroxychinolinen; und wobei das zweite Radioisotop Tc-99m, ¹¹¹In oder ⁶⁷Ga ist; oder
(e) der Gruppe, die aus vernetzter Gelatine, N-[Tris(hydroxymethyl)methyl]acrylamid und Vinylphosphonat besteht; und wobei die anhängenden Einheiten unabhängig aus der Gruppe ausgewählt sind, die aus Phosphonsäuren, Bisphosphonsäuren, Sulfonsäuren und Carbonsäuren besteht; und wobei das zweite Radioisotop Tc-99m, ¹¹¹In oder ⁶⁷Ga ist; oder
(f) der Gruppe, die aus N-[Tris(hydroxymethyl)methyl]acrylamid und Vinylphosphonat besteht; und wobei die anhängenden Einheiten Phosphonsäuren sind; und wobei das zweite Radioisotop Tc-99m, ¹¹¹In oder ⁶⁷Ga ist; oder
(g) der Gruppe, die aus N-[Tris(hydroxymethyl)methyl]acrylamid und Vinylphosphonat besteht; und wobei die anhängenden Einheiten Phosphonsäuren sind; und wobei das zweite Radioisotop ¹¹¹In ist.

8. Mikrosphäre nach Anspruch 2, wobei das Verhältnis der Radioaktivität des zweiten Radioisotops zu dem ersten Radioisotop in dem Bereich von etwa 1 : 10 bis etwa 1 : 10⁷ zum Zeitpunkt der Verwendung oder in dem Bereich von etwa 1 : 10² bis 1 : 10⁶ zum Zeitpunkt der Verwendung oder in dem Bereich von etwa 1 : 10⁴ bis 1 : 10⁵ zum Zeitpunkt der Verwendung oder in dem Bereich von etwa 1 : 10 bis 1 : 10 zum Zeitpunkt der Verwendung liegt.

9. Mikrosphäre nach Anspruch 2, wobei das erste Radioisotop nicht aus der Mikrosphäre in einem Ausmaß von größer als etwa 3 % herausgelöst wird, wobei das zweite Radioisotop nicht aus der Mikrosphäre in einem Ausmaß von größer als etwa 3 % oder in einem Ausmaß von größer als etwa 1 % herausgelöst wird, wobei das zweite Radioisotop nicht aus der Mikrosphäre in einem Ausmaß von größer als etwa 1 % herausgelöst wird.

10. Mikrosphäre nach Anspruch 1 oder 2, wobei die Mikrosphäre weiter Folgendes umfasst: (a) einen biologischen Wirkstoff oder (b) ein kontrastverstärkendes Mittel, optional wobei das kontrastverstärkende Mittel aus der Gruppe ausgewählt ist, die aus Folgenden besteht: röntgenopaken Materialien, paramagnetischen Materialien, schweren Atomen, Übergangsmetallen, Lanthanoiden, Actinoiden und Farbstoffen.

11. Mikrosphäre nach Anspruch 1 oder 2, wobei der Durchmesser der Mikrosphäre in dem Bereich von etwa 1-2000 Mikrometer, etwa 1-1000 Mikrometer, etwa 1-500 Mikrometer, etwa 1-100 Mikrometer oder etwa 10-40 Mikrometer liegt.

12. Verfahren zur Herstellung einer radioaktiven Mikrosphäre, das die folgenden Schritte umfasst:
Vereinigen von Zirconium und einer Mikrosphäre, die ein hydrophiles Polymer umfasst, das eine Vielzahl von anhängenden Einheiten umfasst, wodurch ein Mikrosphären-Metall-Komplex gebildet wird; und
Umwandeln des Zirconiums in dem Mikrosphären-Metall-Komplex zu seinem Oxid, Polyoxometallat, Hydroxid, Alkoxid oder Carboxylat, wodurch eine Metall-markierte Mikrosphäre gebildet wird; und
Vereinigen der Metall-markierten Mikrosphäre mit einem ersten Radioisotop, wobei das Radioisotop in der Form von ³²P-Phosphat vorliegt, wodurch eine radioaktive Metall-markierte Mikrosphäre gebildet wird.

13. Verfahren nach Anspruch 12, wobei das hydrophile Polymer ein oder mehrere polymerisierte Monomere umfasst, die aus Folgenden ausgewählt sind:
(a) der Gruppe, die aus Folgenden besteht: vernetzter Gelatine, oxidierter Stärke, Alginat, Gellan, Gummiarabikum, Galactan, Arabinogalactan, Chitosan, Hyaluronsäure, Chondroitinsulfat, Keratansulfat, Heparansulfat, Dermatansulfat, Carboxymethylcellulose, oxidierter Cellulose, Acrylat, Methacrylat, Ethylenglycolmethacrylatphosphat, Vinylphosphonat, N-[Tris(hydroxymethyl)methyl]acrylamid, N,N'-Methylenbisacrylamid, N',N'-Diallylweinsäurediamid und Glyoxalbisacrylamid; oder
(b) der Gruppe, die aus vernetzter Gelatine, N-[Tris(hydroxymethyl)methyl]acrylamid und Vinylphosphonat besteht.

14. Verfahren nach Anspruch 12 oder 13, wobei die anhängenden Einheiten unabhängig aus Folgenden ausgewählt sind:
(a) der Gruppe, die aus Folgenden besteht: Phosphonsäuren, Bisphosphonsäuren, Phosphaten, Polyphosphaten, Diphosphaten, Triphosphaten, Sulfonsäuren, Sulfaten, Carbonsäuren, Carbaminsäuren, Hydroxamsäuren, Acylhydraziden, Thiolen, Aminen, Silicaten, Aluminaten, Titanaten, Zirconaten, Pyridinen, Imidazolen, Thiophenen, Thiazolen, Furanen, Purinen, Pyrimidinen und Hydroxychinolinen; oder
(b) der Gruppe, die aus Phosphonsäuren, Bisphosphonsäuren, Sulfonsäuren und Carbonsäuren besteht.

15. Verfahren nach Anspruch 12, das die folgenden Schritte umfasst:
Vereinigen von Zirconium und einer Mikrosphäre, die ein hydrophiles Polymer umfasst, das eine Vielzahl von anhängenden Einheiten umfasst, wodurch ein Mikrosphären-Metall-Komplex gebildet wird; wobei das hydrophile Polymer ein oder mehrere polymerisierte Monomere umfasst, die aus der Gruppe ausgewählt sind, die aus vernetzter Gelatine, N-[Tris(hydroxymethyl)methyl]acrylamid und Vinylphosphonat besteht; und wobei die anhängenden Einheiten unabhängig aus der Gruppe ausgewählt sind, die aus Phosphonsäuren, Bisphosphonsäuren, Sulfonsäuren und Carbonsäuren besteht; und
Umwandeln des Zirconiums in dem Mikrosphären-Metall-Komplex zu seinem Oxid, Polyoxometallat, Hydroxid, Alkoxid oder Carboxylat, wodurch eine Metall-markierte Mikrosphäre gebildet wird; und
Vereinigen der Metall-markierten Mikrosphäre mit einem ersten Radioisotop, wobei das erste Radioisotop in der Form von ³²P-Phosphat vorliegt, wodurch eine radioaktive Metall-markierte Mikrosphäre gebildet wird.

16. Verfahren nach Anspruch 12, wobei das hydrophile Polymer ein oder mehrere polymerisierte Monomere umfasst, die aus der Gruppe ausgewählt sind, die aus N-[Tris(hydroxymethyl)methyl]acrylamid und Vinylphosphonat besteht; und wobei die anhängenden Einheiten Phosphonsäuren sind.

17. Verfahren zur Herstellung einer radioaktiven Mikrosphäre, das die folgenden Schritte umfasst:
Vereinigen eines Zirconiumoxids, -polyoxometallats, -hydroxids, -alkoxids oder -carboxylats oder einer Kombination davon und einer Mikrosphäre, die ein hydrophiles Polymer umfasst, das eine Vielzahl von anhängenden Einheiten umfasst, wodurch eine Metall-markierte Mikrosphäre gebildet wird; und
Vereinigen der Metall-markierten Mikrosphäre mit einem ersten Radioisotop, wobei das erste Radioisotop in der Form von ³²P-Phosphat vorliegt, wodurch eine radioaktive Metall-markierte Mikrosphäre gebildet wird.

18. Verfahren nach Anspruch 17, wobei das hydrophile Polymer ein oder mehrere polymerisierte Monomere umfasst, die aus Folgenden ausgewählt sind:
(a) der Gruppe, die aus Folgenden besteht: vernetzter Gelatine, oxidierter Stärke, Alginat, Gellan, Gummiarabikum, Galactan, Arabinogalactan, Chitosan, Hyaluronsäure, Chondroitinsulfat, Keratansulfat, Heparansulfat, Dermatansulfat, Carboxymethylcellulose, oxidierter Cellulose, Acrylat, Methacrylat, Ethylenglycolmethacrylatphosphat, Vinylphosphonat, N-[Tris(hydroxymethyl)methyl]acrylamid, N,N'-Methylenbisacrylamid, N',N'-Diallylweinsäurediamid und Glyoxalbisacrylamid; oder
(b) der Gruppe, die aus N-[Tris(hydroxymethyl)methyl]acrylamid und Vinylphosphonat besteht.

19. Verfahren nach Anspruch 17 oder 18, wobei die anhängenden Einheiten unabhängig aus Folgenden ausgewählt sind:
(a) der Gruppe, die aus Folgenden besteht: Phosphonsäuren, Bisphosphonsäuren, Phosphaten, Polyphosphaten, Diphosphaten, Triphosphaten, Sulfonsäuren, Sulfaten, Carbonsäuren, Carbaminsäuren, Hydroxamsäuren, Acylhydraziden, Thiolen, Aminen, Silicaten, Aluminaten, Titanaten, Zirconaten, Pyridinen, Imidazolen, Thiophenen, Thiazolen, Furanen, Purinen, Pyrimidinen und Hydroxychinolinen; oder
(b) Phosphonsäuren.

20. Verfahren zur Herstellung einer radioaktiven Mikrosphäre, das die folgenden Schritte umfasst:
Bilden einer Mikrosphäre aus einem Polymer, das eine Vielzahl von anhängenden Einheiten umfasst, in Gegenwart von Zirconium, wodurch ein Mikrosphären-Metall-Komplex gebildet wird; und
Umwandeln des Zirconiums in dem Mikrosphären-Metall-Komplex zu seinem Oxid, Polyoxometallat, Hydroxid, Alkoxid oder Carboxylat, wodurch eine Metall-markierte Mikrosphäre gebildet wird; und
Vereinigen der Metall-markierten Mikrosphäre mit einem ersten Radioisotop, wobei das erste Radioisotop in der Form von ³²P-Phosphat vorliegt, wodurch eine radioaktive Metall-markierte Mikrosphäre gebildet wird.

21. Verfahren nach Anspruch 20, wobei das hydrophile Polymer ein oder mehrere polymerisierte Monomere umfasst, die aus Folgenden ausgewählt sind:
(a) der Gruppe, die aus Folgenden besteht: vernetzter Gelatine, oxidierter Stärke, Alginat, Gellan, Gummiarabikum, Galactan, Arabinogalactan, Chitosan, Hyaluronsäure, Chondroitinsulfat, Keratansulfat, Heparansulfat, Dermatansulfat, Carboxymethylcellulose, oxidierter Cellulose, Acrylat, Methacrylat, Ethylenglycolmethacrylatphosphat, Vinylphosphonat, N-[Tris(hydroxymethyl)methyl]acrylamid, N,N'-Methylenbisacrylamid, N',N'-Diallylweinsäurediamid und Glyoxalbisacrylamid; oder
(b) der Gruppe, die aus N-[Tris(hydroxymethyl)methyl]acrylamid und Vinylphosphonat besteht.

22. Verfahren nach Anspruch 20 oder 21, wobei die anhängenden Einheiten unabhängig aus der Gruppe ausgewählt sind, die aus Folgenden besteht: Phosphonsäuren, Bisphosphonsäuren, Phosphaten, Polyphosphaten, Diphosphaten, Triphosphaten, Sulfonsäuren, Sulfaten, Carbonsäuren, Carbaminsäuren, Hydroxamsäuren, Acylhydraziden, Thiolen, Aminen, Silicaten, Aluminaten, Titanaten, Zirconaten, Pyridinen, Imidazolen, Thiophenen, Thiazolen, Furanen, Purinen, Pyrimidinen und Hydroxychinolinen.

23. Verfahren nach einem der Ansprüche 12 bis 22, wobei die Mikrosphäre mit einem zweiten Radioisotop vereinigt wird und wobei die Atomzahl des ersten Radioisotops nicht die gleiche ist wie die Atomzahl des zweiten Radioisotops.

24. Verfahren nach einem der Ansprüche 12 bis 23, wobei die Mikrosphären für die Verabreichung unter Verwendung eines Katheters oder einer Spritze geeignet sind.

25. Radioaktive Metall-markierte Mikrosphäre nach einem der Ansprüche 1-11, wobei das hydrophile Polymer ein oder mehrere polymerisierte Monomere umfasst, die aus der Gruppe von N-[Tris(hydroxymethyl)methyl]acrylamid und Vinylphosphonat ausgewählt sind, und wobei die anhängenden Einheiten Phosphonsäuren sind, für die Behandlung eines Säugers, der an Folgendem leidet: einer Kopferkrankung, einer Halserkrankung, einer Thoraxerkrankung, einer Abdominalerkrankung, einer Beckenerkrankung, einem Krebs, chronischer hämophiler Synovitis oder Arthritis.

26. Mikrosphäre nach einem der Ansprüche 1 bis 11 für die Behandlung von Krebs, Synovektomie oder Arthritis.

## Revendications

1. Microsphère, comprenant un polymère hydrophile comprenant une pluralité de motifs pendants ; un oxyde, polyoxométallate, hydroxyde, alcoxyde, carboxylate de zirconium, ou une combinaison de ceux-ci ; et un premier radioisotope, où ledit premier radioisotope se trouve sous la forme de phosphate ³²P.

2. Microsphère selon la revendication 1, comprenant en outre un deuxième radioisotope ; où le nombre atomique du premier radioisotope n'est pas identique au nombre atomique du deuxième radioisotope.

3. Microsphère selon la revendication 1 ou 2, dans laquelle ledit polymère hydrophile comprend un ou plusieurs monomères polymérisés choisis dans
(a) le groupe constitué par les composés acryliques, les composés vinyliques, les acétals, les composés allyliques, les composés cellulosiques, les méthacrylates, les polyamides, un polycarbonate, les polyesters, un polyimide, les polyoléfines, les polyphosphates, les polyuréthanes, les silicones, les composés styréniques, et les polysaccharides ;
(b) le groupe constitué par la gélatine réticulée, l'amidon oxydé, un alginate, une gomme gellane, une gomme arabique, un galactane, un arabinogalactane, un chitosane, un hyaluronane, un sulfate de chondroïtine, un sulfate de kératane, un sulfate d'héparane, un sulfate de dermatane, une carboxyméthylcellulose, une cellulose oxydée, un acrylate, un méthacrylate, un phosphate de méthacrylate d'éthylène glycol, le phosphonate de vinyle, le N-[tris(hydroxyméthyl)méthyl]acrylamide, le N,N'-méthylène-bis-acrylamide, le N',N'-diallyltartardiamide, et le glyoxal-bis-acrylamide ;
(c) le groupe constitué par la gélatine réticulée, le N-[tris(hydroxy-méthyl)méthyl]acrylamide et le phosphonate de vinyle ; ou
(d) le groupe constitué par le N-[tris(hydroxyméthyl)-méthyl]acrylamide et le phosphonate de vinyle.

4. Microsphère selon la revendication 1 ou 2, dans laquelle lesdits motifs pendants sont choisis indépendamment dans
(a) le groupe constitué par les acides phosphoniques, les phosphates, les acides bisphosphoniques, les polyphosphates, les diphosphates, les triphosphates, les acides sulfoniques, les sulfates, les acides carboxyliques, les acides carbamiques, les acides hydroxamiques, les hydrazides d'acyle, les thiols, les aminés, les silicates, les aluminates, les titanates, les zirconates, les pyridines, les imidazoles, les thiophènes, les thiazoles, les furanes, les purines, les pyrimidines, et les hydroxyquinoléines ;
(b) le groupe constitué par les acides phosphoniques, les acides bisphosphoniques, les acides sulfoniques, et les acides carboxyliques ; ou
(c) les acides phosphoniques.

5. Microsphère selon la revendication 2, dans laquelle ledit deuxième radioisotope est (a) Tc-99m, ¹¹¹In ou ⁶⁷Ga ; ou (b) ¹¹¹In.

6. Microsphère selon la revendication 2, dans laquelle ledit deuxième radioisotope est ¹¹¹In.

7. Microsphère selon la revendication 1 ou 2, dans laquelle ledit polymère hydrophile comprend un ou plusieurs monomères polymérisés, choisis dans
(a) le groupe constitué par la gélatine réticulée, l'amidon oxydé, un alginate, une gomme gellane, une gomme arabique, un galactane, un arabinogalactane, un chitosane, un hyaluronane, un sulfate de chondroïtine, un sulfate de kératane, un sulfate d'héparane, un sulfate de dermatane, une carboxyméthylcellulose, une cellulose oxydée, un acrylate, un méthacrylate, un phosphate de méthacrylate d'éthylène glycol, le phosphonate de vinyle, le N-[tris(hydroxyméthyl)méthyl]acrylamide, le N,N'-méthylène-bis-acrylamide, le N',N'-diallyltartardiamide, et le glyoxal-bis-acrylamide ; et où lesdits motifs pendants sont choisis indépendamment dans le groupe constitué par les acides phosphoniques, les acides bisphosphoniques, les phosphates, les polyphosphates, les diphosphates, les triphosphates, les acides sulfoniques, les sulfates, les acides carboxyliques, les acides carbamiques, les acides hydroxamiques, les hydrazides d'acyle, les thiols, les aminés, les silicates, les aluminates, les titanates, les zirconates, les pyridines, les imidazoles, les thiophènes, les thiazoles, les furanes, les purines, les pyrimidines, et les hydroxyquinoléines ; ou
(b) le groupe constitué par la gélatine réticulée, le N-[tris(hydroxyméthyl)-méthyl]acrylamide et le phosphonate de vinyle ; et où lesdits motifs pendants sont choisis indépendamment dans le groupe constitué par les acides phosphoniques, les acides bisphosphoniques, les acides sulfoniques, et les acides carboxyliques ; ou
(c) le groupe constitué par le N-[tris(hydroxyméthyl)méthyl]acrylamide et le phosphonate de vinyle ; et où lesdits motifs pendants sont des acides phosphoniques ; ou
(d) le groupe constitué par la gélatine réticulée, l'amidon oxydé, un alginate, une gomme gellane, une gomme arabique, un galactane, un arabinogalactane, un chitosane, un hyaluronane, un sulfate de chondroïtine, un sulfate de kératane, un sulfate d'héparane, un sulfate de dermatane, une carboxyméthylcellulose, une cellulose oxydée, un acrylate, un méthacrylate, un phosphate de méthacrylate d'éthylène glycol, le phosphonate de vinyle, le N-[tris(hydroxyméthyl)méthyl]acrylamide, le N,N'-méthylène-bis-acrylamide, le N',N'-diallyltartardiamide, et le glyoxal-bis-acrylamide ; et où lesdits motifs pendants sont choisis indépendamment dans le groupe constitué par les acides phosphoniques, les acides bisphosphoniques, les phosphates, les polyphosphates, les diphosphates, les triphosphates, les acides sulfoniques, les sulfates, les acides carboxyliques, les acides carbamiques, les acides hydroxamiques, les hydrazides d'acyle, les thiols, les aminés, les silicates, les aluminates, les titanates, les zirconates, les pyridines, les imidazoles, les thiophènes, les thiazoles, les furanes, les purines, les pyrimidines, et les hydroxyquinoléines; et où ledit deuxième radioisotope est Tc-99m, ¹¹¹In ou ⁶⁷Ga ; ou
(e) le groupe constitué par la gélatine réticulée, le N-[tris(hydroxyméthyl)-méthyl]acrylamide et le phosphonate de vinyle ; et où lesdits motifs pendants sont choisis indépendamment dans le groupe constitué par les acides phosphoniques, les acides bisphosphoniques, les acides sulfoniques, et les acides carboxyliques ; et où le deuxième radioisotope est Tc-99m, ¹¹¹In ou ⁶⁷Ga ; ou
(f) le groupe constitué par le N-[tris(hydroxyméthyl)-méthyl]acrylamide et le phosphonate de vinyle ; et où lesdits motifs pendants sont des acides phosphoniques ; et où ledit deuxième radioisotope est Tc-99m, ¹¹¹In ou ⁶⁷Ga ; ou
(g) le groupe constitué par le N-[tris(hydroxyméthyl)-méthyl]acrylamide et le phosphonate de vinyle ; et où lesdits motifs pendants sont des acides phosphoniques ; et où ledit deuxième radioisotope est ¹¹¹In.

8. Microsphère selon la revendication 2, dans laquelle le rapport de la radioactivité du deuxième radioisotope au premier radioisotope se trouve dans la plage allant d'environ 1:10 à environ 1:10⁷ au moment de l'utilisation ; ou dans la plage allant d'environ 1:10² à 1:10⁶ au moment de l'utilisation ; ou dans la plage allant d'environ 1:10⁴ à 1:10⁵ au moment de l'utilisation ; ou dans la plage allant d'environ 1:10 à 1:10³ au moment de l'utilisation.

9. Microsphère selon la revendication 2, dans laquelle ledit premier radioisotope n'est pas lixivié hors de ladite microsphère jusqu'à un taux supérieur à environ 3% ; où ledit deuxième radioisotope n'est pas lixivié hors de ladite microsphère jusqu'à un taux supérieur à environ 3% ; ou jusqu'à un taux supérieur à environ 1% ; où ledit deuxième radioisotope n'est pas lixivié hors de ladite microsphère jusqu'à un taux supérieur à environ 1%.

10. Microsphère selon la revendication 1 ou 2, où ladite microsphère comprend en outre (a) un agent biologiquement actif ; ou (b) un agent d'amélioration de contraste ; éventuellement où ledit agent d'amélioration de contraste est choisi dans le groupe constitué par les matériaux radio-opaques, les matériaux paramagnétiques, les atomes lourds, les métaux de transition, les lanthanides, les actinides, et les colorants.

11. Microsphère selon la revendication 1 ou 2, dans laquelle le diamètre de ladite microsphère se trouve dans la plage d'environ 1-2000 micromètres, d'environ 1-1000 micromètres, d'environ 1-500 micromètres, d'environ 1-100 micromètres, ou d'environ 10-40 micromètres.

12. Méthode de préparation d'une microsphère radioactive, comprenant les étapes consistant à :
combiner du zirconium et une microsphère comprenant un polymère hydrophile comprenant une pluralité de motifs pendants, formant ainsi un complexe microsphère-métal ; et
convertir le zirconium dans le complexe microsphère-métal en son oxyde, polyoxométallate, hydroxyde, alcoxyde ou carboxylate, formant ainsi une microsphère à marquage métallique ; et
combiner ladite microsphère à marquage métallique avec un premier radioisotope, où ledit radioisotope se trouve sous la forme de phosphate ³²P, formant ainsi une microsphère à marquage métallique radioactive.

13. Méthode selon la revendication 12, dans laquelle ledit polymère hydrophile comprend un ou plusieurs monomères polymérisés, choisis dans
(a) le groupe constitué par la gélatine réticulée, l'amidon oxydé, un alginate, une gomme gellane, une gomme arabique, un galactane, un arabinogalactane, un chitosane, un hyaluronane, un sulfate de chondroïtine, un sulfate de kératane, un sulfate d'héparane, un sulfate de dermatane, une carboxyméthylcellulose, une cellulose oxydée, un acrylate, un méthacrylate, un phosphate de méthacrylate d'éthylène glycol, le phosphonate de vinyle, le N-[tris(hydroxyméthyl)méthyl]acrylamide, le N,N'-méthylène-bis-acrylamide, le N',N'-diallyltartardiamide, et le glyoxal-bis-acrylamide ; ou
(b) le groupe constitué par la gélatine réticulée, le N-[tris(hydroxyméthyl)-méthyl]acrylamide et le phosphonate de vinyle.

14. Méthode selon la revendication 12 ou 13, dans laquelle lesdits motifs pendants sont choisis indépendamment dans
(a) le groupe constitué par les acides phosphoniques, les acides bisphosphoniques, les phosphates, les polyphosphates, les diphosphates, les triphosphates, les acides sulfoniques, les sulfates, les acides carboxyliques, les acides carbamiques, les acides hydroxamiques, les hydrazides d'acyle, les thiols, les amines, les silicates, les aluminates, les titanates, les zirconates, les pyridines, les imidazoles, les thiophènes, les thiazoles, les furanes, les purines, les pyrimidines, et les hydroxyquinoléines ; ou
(b) le groupe constitué par les acides phosphoniques, les acides bisphosphoniques, les acides sulfoniques, et les acides carboxyliques.

15. Méthode selon la revendication 12, comprenant les étapes consistant à :
combiner du zirconium et une microsphère comprenant un polymère hydrophile comprenant une pluralité de motifs pendants, formant ainsi un complexe microsphère-métal ; où ledit polymère hydrophile comprend un ou plusieurs monomères polymérisés choisis dans le groupe constitué par la gélatine réticulée, le N-[tris(hydroxyméthyl)-méthyl]acrylamide et le phosphonate de vinyle ; et où lesdits motifs pendants sont choisis indépendamment dans le groupe constitué par les acides phosphoniques, les acides bisphosphoniques, les acides sulfoniques, et les acides carboxyliques ; et
convertir le zirconium dans le complexe microsphère-métal en son oxyde, polyoxométallate, hydroxyde, alcoxyde ou carboxylate, formant ainsi une microsphère à marquage métallique ; et
combiner ladite microsphère à marquage métallique avec un premier radioisotope, où ledit premier radioisotope se trouve sous la forme de phosphate ³²P, formant ainsi une microsphère à marquage métallique radioactive.

16. Méthode selon la revendication 12, dans laquelle ledit polymère hydrophile comprend un ou plusieurs monomères polymérisés choisis dans le groupe constitué par le N-[tris(hydroxyméthyl)-méthyl]acrylamide et le phosphonate de vinyle ; et où lesdits motifs pendants sont des acides phosphoniques.

17. Méthode de préparation d'une microsphère radioactive, comprenant les étapes consistant à :
combiner un oxyde, polyoxométallate, hydroxyde, alcoxyde ou carboxylate de zirconium, ou une combinaison de ceux-ci, et une microsphère comprenant un polymère hydrophile comprenant une pluralité de motifs pendants, formant ainsi un complexe microsphère-métal ; et
combiner ladite microsphère à marquage métallique avec un premier radioisotope, où ledit premier radioisotope se trouve sous la forme de phosphate ³²P, formant ainsi une microsphère à marquage métallique radioactive.

18. Méthode selon la revendication 17, dans laquelle ledit polymère hydrophile comprend un ou plusieurs monomères polymérisés choisis dans
(a) le groupe constitué par la gélatine réticulée, l'amidon oxydé, un alginate, une gomme gellane, une gomme arabique, un galactane, un arabinogalactane, un chitosane, un hyaluronane, un sulfate de chondroïtine, un sulfate de kératane, un sulfate d'héparane, un sulfate de dermatane, une carboxyméthylcellulose, une cellulose oxydée, un acrylate, un méthacrylate, un phosphate de méthacrylate d'éthylène glycol, le phosphonate de vinyle, le N-[tris(hydroxyméthyl)méthyl]acrylamide, le N,N'-méthylène-bis-acrylamide, le N',N'-diallyltartardiamide, et le glyoxal-bis-acrylamide ; ou
(b) le groupe constitué par le N-[tris(hydroxyméthyl)-méthyl]acrylamide et le phosphonate de vinyle.

19. Méthode selon la revendication 17 ou 18, dans laquelle lesdits motifs pendants sont choisis indépendamment dans
(a) le groupe constitué par les acides phosphoniques, les acides bisphosphoniques, les phosphates, les polyphosphates, les diphosphates, les triphosphates, les acides sulfoniques, les sulfates, les acides carboxyliques, les acides carbamiques, les acides hydroxamiques, les hydrazides d'acyle, les thiols, les amines, les silicates, les aluminates, les titanates, les zirconates, les pyridines, les imidazoles, les thiophènes, les thiazoles, les furanes, les purines, les pyrimidines, et les hydroxyquinoléines ; ou
(b) les acides phosphoniques.

20. Méthode de préparation d'une microsphère radioactive, comprenant les étapes consistant à :
former une microsphère à partir d'un polymère comprenant une pluralité de motifs pendants en présence de zirconium, formant ainsi un complexe microsphère-métal ; et
convertir le zirconium dans le complexe microsphère-métal en son oxyde, polyoxométallate, hydroxyde, alcoxyde ou carboxylate, formant ainsi une microsphère à marquage métallique ; et
combiner ladite microsphère à marquage métallique avec un premier radioisotope, où ledit premier radioisotope se trouve sous la forme de phosphate ³²P, formant ainsi une microsphère à marquage métallique radioactive.

21. Méthode selon la revendication 20, dans laquelle ledit polymère hydrophile comprend un ou plusieurs monomères polymérisés choisis dans
(a) le groupe constitué par la gélatine réticulée, l'amidon oxydé, un alginate, une gomme gellane, une gomme arabique, un galactane, un arabinogalactane, un chitosane, un hyaluronane, un sulfate de chondroïtine, un sulfate de kératane, un sulfate d'héparane, un sulfate de dermatane, une carboxyméthylcellulose, une cellulose oxydée, un acrylate, un méthacrylate, un phosphate de méthacrylate d'éthylène glycol, le phosphonate de vinyle, le N-[tris(hydroxyméthyl)méthyl]acrylamide, le N,N'-méthylène-bis-acrylamide, le N',N'-diallyltartardiamide, et le glyoxal-bis-acrylamide ; ou
(b) le groupe constitué par le N-[tris(hydroxyméthyl)-méthyl]acrylamide et le phosphonate de vinyle.

22. Méthode selon la revendication 20 ou 21, dans laquelle lesdits motifs pendants sont choisis indépendamment dans le groupe constitué par les acides phosphoniques, les acides bisphosphoniques, les phosphates, les polyphosphates, les diphosphates, les triphosphates, les acides sulfoniques, les sulfates, les acides carboxyliques, les acides carbamiques, les acides hydroxamiques, les hydrazides d'acyle, les thiols, les amines, les silicates, les aluminates, les titanates, les zirconates, les pyridines, les imidazoles, les thiophènes, les thiazoles, les furanes, les purines, les pyrimidines, et les hydroxyquinoléines.

23. Méthode selon l'une quelconque des revendications 12 à 22, dans laquelle ladite microsphère est combinée avec un deuxième radioisotope et où le nombre atomique du premier radioisotope n'est pas identique au nombre atomique du deuxième radioisotope.

24. Méthode selon l'une quelconque des revendications 12 à 23 , dans laquelle lesdites microsphères sont convenables pour une administration à l'aide d'un cathéter ou d'une seringue.

25. Microsphère à marquage métallique radioactive selon l'une quelconque des revendications 1-11, dans laquelle ledit polymère hydrophile comprend un ou plusieurs monomères polymérisés choisis dans le groupe constitué par le N-[tris(hydroxyméthyl)-méthyl]acrylamide et le phosphonate de vinyle ; et où lesdits motifs pendants sont des acides phosphoniques ; pour le traitement d'un mammifère souffrant d'un trouble de la tête, d'un trouble du cou, d'un trouble du thorax, d'un trouble abdominal, d'un trouble du pelvis, d'un cancer, d'une synovite hémophilique chronique, ou d'arthrite.

26. Microsphère selon l'une quelconque des revendications 1-11, pour le traitement d'un cancer, d'une synovectomie, ou de l'arthrite.
